(19) Europäisches Patentamt
European Patent Office
Office européen des brevets

(11) EP 4 163 922 A1

(12) EUROPEAN PATENT APPLICATION

(43) Date of publication:
**12.04.2023 Bulletin 2023/15**

(21) Application number: **21201799.0**

(22) Date of filing: **10.10.2021**

(51) International Patent Classification (IPC):
***G16H 10/20*** *(2018.01)* ***G16H 50/20*** *(2018.01)*
***G16H 50/70*** *(2018.01)*

(52) Cooperative Patent Classification (CPC):
**G16H 50/20; G16H 10/20; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Centre Hospitalier Universitaire Vaudois (CHUV)**
**1011 Lausanne (CH)**

(72) Inventors:
- **Mansouri, Nahal**
  **1011 Lausanne (CH)**
- **Asgary, Amirhossein**
  **Tehran (IR)**
- **Jamshidi, Elham**
  **Tehran (IR)**

(74) Representative: **Vossius & Partner Patentanwälte Rechtsanwälte mbB**
**Siebertstraße 3**
**81675 München (DE)**

(54) **A METHOD FOR PREDICTING SIDE EFFECTS OF A CORONAVIRUS VACCINE**

(57) The present invention relates to a method for predicting side effects of a coronavirus vaccine and to a method for selecting a subject qualifying for vaccination with a coronavirus vaccine. Instant methods are particularly useful wherein the coronavirus vaccine is a SARS-CoV2 vaccine

EP 4 163 922 A1

## Description

The present invention relates to a method for predicting side effects of a coronavirus vaccine and to a method for selecting a subject qualifying for vaccination with a coronavirus vaccine. Instant methods are particularly useful wherein the coronavirus vaccine is a SARS-CoV2 vaccine.

The ongoing COVID-19 pandemics, which could not be controlled despite applied measures including strict personal hygiene guidelines and social distancing, necessitated mass population vaccination as soon as effective vaccines have become available.[1] This has been enabled by the WHO Emergency Use Listing (EUL) issuance, designating the COVID-19 approved vaccines.[2] While widely recognized as one of the most potent weapons against many infectious diseases, the potential adverse side effects of vaccines may slow down or prevent the progress of the population vaccination campaign in certain populations.[3]

Vaccine adverse effects are correlated with the activity of the immune system, which in turn is closely related to sex, age, medical background and underlying disorders, and drug history.[4] In 2018 Kopsaftis Z et al. reported enhanced injection site side effects of influenza vaccines in elderly and Chronic obstructive pulmonary disease (COPD) patients.[5] Immunocompromised patients with primary immunodeficiency and hematological malignancies might be susceptible to vaccine-derived infections and stronger levels of adverse effects.[6, 7]

Recognizing, anticipating, and predicting the adverse side effects of all sorts of vaccines including COVID-19 vaccines can help recipients better understand their body's immune response to the vaccination process and decrease their anxiety about the adverse effects. These predictions can pave the way for the next steps which would be personalized vaccinology.[8]

In the case of the COVID-19 pandemic, the general public's perception of the vaccines' adverse reactions might negatively influence the willingness for vaccination, and ultimately disrupt the goal of herd immunity within the population.[9]

There is an urgent need to predict and clarify the vaccines' adverse effects on individuals. Based on the potential correlation between health-related traits and adverse side effects of vaccines, applying the medical and personal records of vaccine recipients may support a personalized estimate of each individual's immune response and adverse effects after vaccination.

Document AU 2020/102250 discloses certain COVID-19 early warning system for senior citizens.

Document AU 2020/101336 discloses certain machine learning technique for tracking the COVID-19 patients and their primary and secondary contacts to prevent the spread of COVID-19.

Document EU2601197C2 discloses certain software system for predicting results of the treatment.

Document US/92/35686 discloses certain methods for using adverse event data to predict types of side effects of drugs based on its supposed molecular interactions. Document CN111768873A discloses certain solution for predicting real-time risk of COVID-19 based on epidemiological findings.

The objective technical problem of the present invention is to provide a method for predicting side effects of a coronavirus in a subject.

The objective technical problem is solved by the embodiments described herein and as characterized by the claims.

The present inventors have surprisingly found that the medical and personal records of vaccine recipients can significantly support a personalized estimate of each individual's immune response and adverse effects after vaccination.

It has been further unexpectedly found by the present inventors that age had the highest negative influence on the probability of adverse side effects to occur among the factors studied by the present inventors. Thus, the probability of the side effects occurrence is inversely proportional to the age of a subject as younger individuals have more intense immune responses, and thus witness more vaccine side effects. The present inventors have further established the course of the past coronavirus disease, in particular COVID-19 infection as the second most influential factor. In other words, the subjects with a history of severe COVID-19 infection experienced more severe vaccine-related adverse effects. It has been further established that severe vaccine-related adverse effects were visible in subjects with a positive past medical history of cancer.

The present invention addresses the unmet medical need and thereby shall help the healthcare authorities worldwide to select the safest vaccine for subjects considering the predicted side effects that every individual subject would experience because of various coronavirus, herein COVID-19 vaccine types. The method of the present invention can make vaccine allocation smarter and safer for the general public. In other words this invention provides a sorting mechanism for the COVID-19 vaccines based on their predicted adverse side effects for every individual. This process can ultimately help and encourage unvaccinated individuals to undergo vaccination.

The invention will be summarized in the following embodiments.

In a first embodiment, the present invention relates to a method for predicting side effects of a coronavirus vaccine in a subject, the method comprising (a) providing subject-specific health and life-style data; and (b) predicting the side effects of a coronavirus vaccine in a subject based on the subject-specific health and life-style data using a

previously trained mathematical model.

In a particular embodiment, the present invention relates to the method for predicting side effects of a coronavirus vaccine in a subject, wherein the subject-specific health and life-style data comprise subject physical parameters, course of the past coronavirus disease, data on medication(s) administered to the subject, medical background, and/or life style data, preferably wherein the subject-specific health and life-style data is collected by using a questionnaire.

In a further particular embodiment, the present invention relates to the method for predicting side effects of a coronavirus vaccine in a subject, wherein the subject physical parameters comprise weight, height, BMI, blood group, age, and/or sex.

In again a further particular embodiment, the present invention relates to the method for predicting side effects of a coronavirus vaccine in a subject, wherein the course of the past coronavirus disease comprises data on severity, vertigo, sore throat, headache, chest pain, feeling paralyzed, loss of consciousness, breathing difficulties, loss of smell, digestive difficulties, cough, pain, fatigue and/or fever.

In again a further particular embodiment, the present invention relates to the method for predicting side effects of a coronavirus vaccine in a subject, wherein the data on medication(s) administered to the subject comprise data on immunosuppressive therapy, chemotherapy, steroids (preferably cortone), respiratory spray, and/or hormone drugs.

In again a further particular embodiment, the present invention relates to the method for predicting side effects of a coronavirus vaccine in a subject, wherein the medical background comprises the data on pregnancy, allergy, psychological issues, skeletal system, liver diseases, kidney diseases, digestive tract diseases, blood diseases, immune system diseases, lung diseases, neurological diseases, active cancer diseases, past history of cancer disease, hypertension, heart disease, and/or diabetes. In again a further particular embodiment, the present invention relates to the method for predicting side effects of a coronavirus vaccine in a subject, wherein the life-style data comprise the data on alcohol consumption, use of narcotics, and/or smoking.

In again a further particular embodiment, the present invention relates to the method for predicting side effects of a coronavirus vaccine in a subject, wherein the subject-specific health and life-style data further comprise data on side effects of a previous dose of a coronavirus vaccine.

In again a further particular embodiment, the present invention relates to the method for predicting side effects of a coronavirus vaccine in a subject, wherein the data on side effects of the previous dose of a coronavirus vaccine comprise data on muscle pain, join paint, chills, nausea, headache, fatigue, fever and/or local side effects.

In again a further particular embodiment, the present invention relates to the method for predicting side effects of a coronavirus vaccine in a subject, wherein the previously trained mathematical model is a machine learning model.

In again a further particular embodiment, the present invention relates to the method for predicting side effects of a coronavirus vaccine in a subject, wherein the machine learning model is selected from Logistic Regression (LR), Random Forest (RF), Multi-Layer Perceptron (MLP), K-Nearest Neighbors (KNN), Support Vector Machine (SVM), and Gradient Boosted Decision Trees (XGBoost).

In again a further particular embodiment, the present invention relates to the method for predicting side effects of a coronavirus vaccine in a subject, wherein the machine learning model is Logistic Regression (LR).

In again a further particular embodiment, the present invention relates to the method for predicting side effects of a coronavirus vaccine in a subject, wherein the previously trained mathematical model has been trained using hyperparameter tuning.

In again a further particular embodiment, the present invention relates to the method for predicting side effects of a coronavirus vaccine in a subject, wherein the predicted side effects of administering a vaccine to a subject include fever, fatigue, headache, nausea, chills, joint pain, muscle pain and/or local side effects.

In again a further particular embodiment, the present invention relates to the method for predicting side effects of a coronavirus vaccine in a subject, wherein the method further comprises the step of (a') training of the previously trained mathematical model. In again a further particular embodiment, the present invention relates to the method for predicting side effects of a coronavirus vaccine in a subject, wherein the previously trained mathematical model is trained using the database comprising subject-specific data on side effects of a coronavirus vaccine in a subject and subject-specific health and life-style data, wherein the database has been compiled for subjects that were previously vaccinated with a coronavirus vaccine.

In again a further particular embodiment, the present invention relates to the method for predicting side effects of a coronavirus vaccine in a subject, wherein the data on side effects of a coronavirus vaccine in a subject is as described herein.

In again a further particular embodiment, the present invention relates to the method for predicting side effects of a coronavirus vaccine in a subject, wherein the subject-specific health and life-style data are as described herein.

In again a further particular embodiment, the present invention relates to the method for predicting side effects of a coronavirus vaccine in a subject, wherein the previously trained mathematical model relies on predictors selected from subject-specific health and life-style data, wherein the subject specific health and life-style data are as described herein.

In again a further particular embodiment, the present invention relates to the method for predicting side effects of a coronavirus vaccine in a subject, wherein the coronavirus vaccine is selected from DNA vaccine, RNA vaccine, adenovirus vector vaccine, inactivated virus vaccine, and subunit vaccine.

In again a further particular embodiment, the present invention relates to the method for predicting side effects of a coronavirus vaccine in a subject, wherein the predicted side effects of a coronavirus vaccine are provided in a form of a subject-specific factsheet.

In a further embodiment, the present invention relates to a method for selecting a subject qualifying for vaccination with a coronavirus vaccine, the method comprising (a) predicting the side effects of a coronavirus vaccine in a subject using the method according to any one of claims 1 to 21, and (b) selecting a subject qualifying for vaccination with a coronavirus vaccine based on predicted side effects of a coronavirus vaccine in said subject.

In a particular embodiment, the method for predicting side effects of a coronavirus vaccine in a subject or the method for selecting a subject qualifying for vaccination with a coronavirus vaccine relates to an embodiment, wherein the coronavirus is SARS-CoV2.

## Brief description of Figures

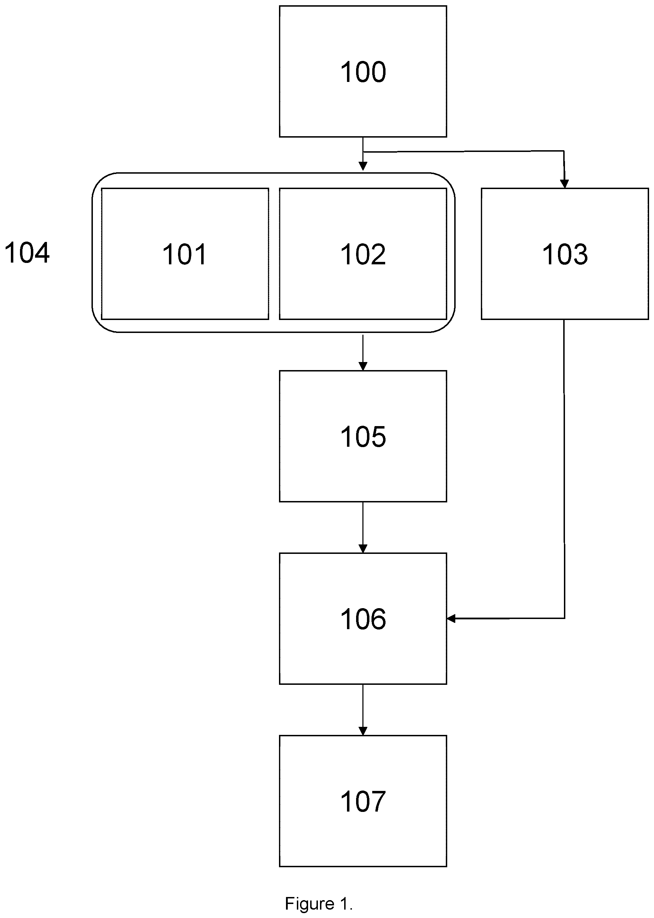

**Figure 1** presents the flow diagram of the incorporation of data for designing and improving machine learning and statistical analysis techniques.

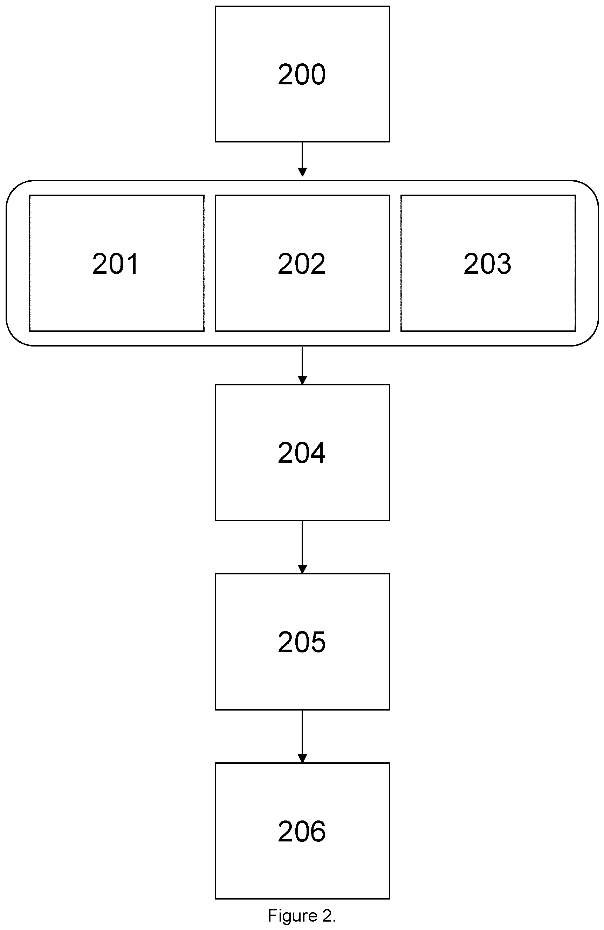

**Figure 2** presents the flow diagram of the machine learning and statistical analysis technique for end users.

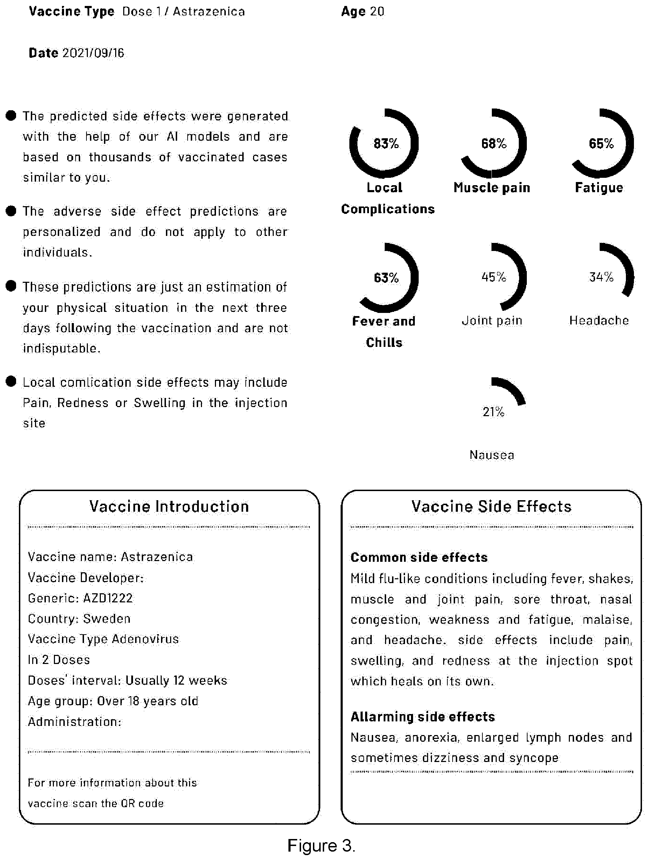

**Figure 3** presents an example of a personalized fact sheet, which is one of the applications of the invention. The Figure provides a possibility for each predicted adverse side effect between 0 and 1. It may also consist of other popular and typical parts of fact sheet, such as a brief description of the administrated vaccine.

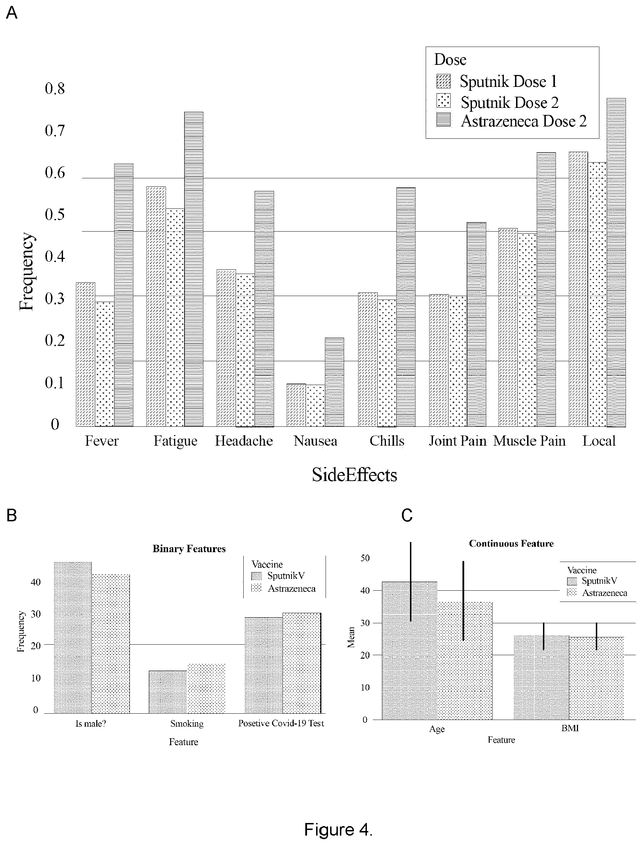

**Figure 4** presents the description of side effects and predictors features that have been used as input data to the models: A) frequency of occurrence of each side effect for first and second dose of a Covid-19 vaccine. B) frequency of binary features including sex, smoking and prior Covid-19 infection. C) distribution of continuous features including age and BMI, error bars shows standard deviation.

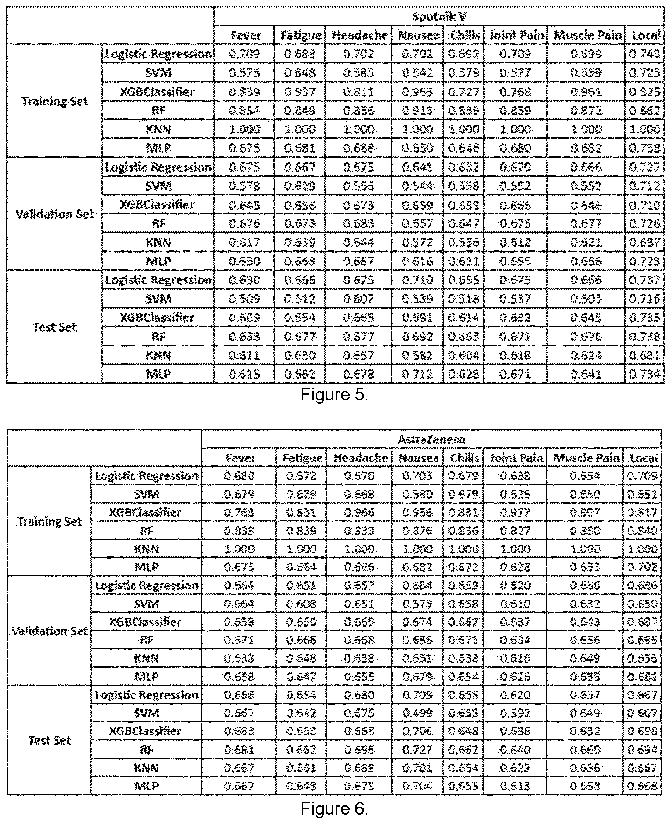

**Figure 5** presents performance of different models for the side effect prediction for the first dose of Sputnik V vaccine. Once the best hyperparameter is found for each model type for each side effect, model average performance is calculated on training, validation and test sets by using ROC-AUC parameter.

**Figure 6** presents performance of different models for the side effect prediction for the first dose of AstraZeneca vaccine. Once the best hyperparameter is found for each model type for each side effect, model average performance is calculated on training, validation and test sets by using ROC-AUC parameter.

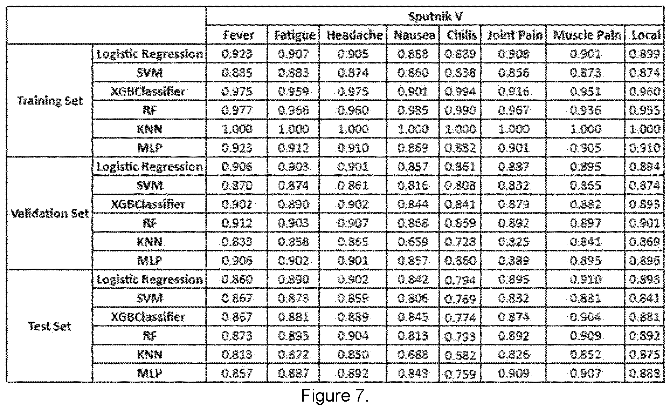

**Figure 7** presents performance of different models for the side effect prediction for the second dose of Sputnik V vaccine. Once the best hyperparameter is found for each model type for each side effect, model average performance is calculated on training, validation and test sets by using ROC-AUC parameter.

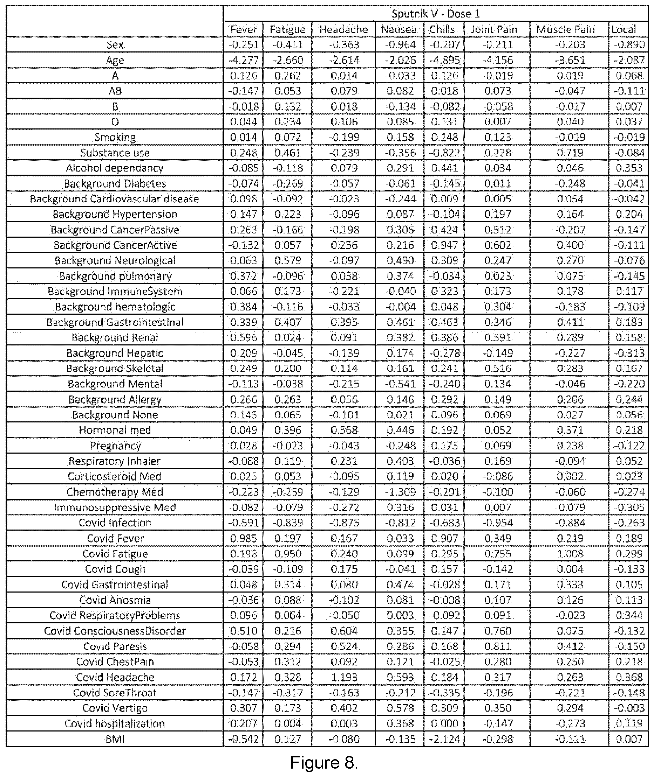

**Figure 8** presents contribution of each feature to the side effect prediction for the first dose of Sputnik V vaccine. The Logistic regression model with the hyperparameter that has been found using hyperparameter tuning has been trained on a training set.

To show each feature contribution to the final outcome Logistic regression coefficient was used. Negative coefficient means that feature has decreasing effect on the side effect probability. The positive coefficient shows features with increasing effect on the probability. In other words, the higher the numbers the more impact it had on the side effect probability.

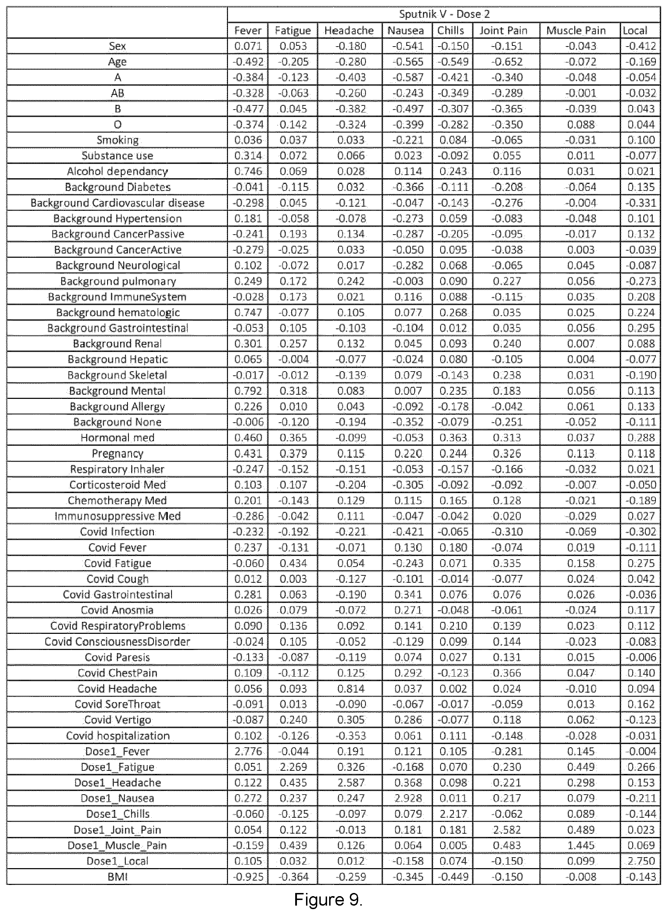

**Figure 9** presents contribution of each feature to the side effect prediction for the second dose of Sputnik V vaccine. The feature set additionally comprises the side effects of the first dose (see Figure 8 hereinabove), which is positively correlated with side effects for the second dose of the vaccine. The Logistic regression model with the hyperparameter that has been found using hyperparameter tuning has been trained on a training set. To show each feature contribution to the final outcome Logistic regression coefficient was used. Negative coefficient means that feature has decreasing effect on the side effect probability. The positive coefficient shows features with increasing effect on the probability. In other words, the higher the numbers the more impact it had on the side effect probability.

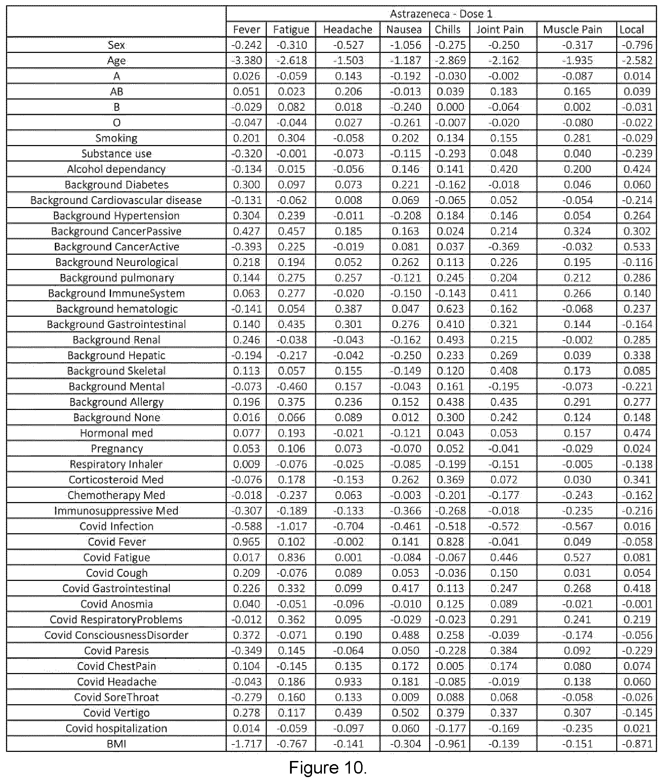

**Figure 10** presents contribution of each feature to the side effect prediction for the first dose of AstraZeneca vaccine. The Logistic regression model with the hyperparameter that has been found using hyperparameter tuning has been trained on a training set. To show each feature contribution to the final outcome Logistic regression coefficient was used. Negative coefficient means that feature has decreasing effect on the side effect probability. The positive coefficient shows features with increasing effect on the probability. In other words, the higher the numbers the more impact it had on the side effect probability.

The embodiments of the present invention will be described in the following. It is to be understood that all possible combinations of features are encompassed.

In one embodiment, the present invention relates to a method for predicting side effects of a coronavirus vaccine in a subject. The method of the present invention comprises the following steps:

(a) providing subject-specific health and life-style data; and
(b) predicting the side effects of a coronavirus vaccine in a subject based on the subject-specific health and life-style data using a previously trained mathematical model.

The subject, as understood herein, is preferably a human subject.

The method for predicting side effects of a coronavirus vaccine in a subject comprises step (a) of providing subject-specific health and life-style data. The invention is not limited with respect of how the subject-specific health and life-style data are collected and any means of collecting the said data from the subject as can be envisaged by the skilled person is encompassed by the present invention. Preferably, as encompassed by the present invention, the subject-specific health and life-style data is collected by using a questionnaire. Any type of questionnaire as to be envisaged by the skilled person can be used herein. For example, questionnaire may be in a paper form to be filled by the subject, or questionnaire may be filled by the subject as an online form. In certain embodiments, the said questionnaire may be filled by a third party, for example by a physician or by a nurse, upon direct interview with the subject.

As understood herein, preferably within the scope of the present invention, the subject specific health and life-style data are represented by features that may be binary features, discrete features or continuous features. Preferably, unless otherwise stated, the features representing the subject specific health and life-style data are binary features. As understood herein, a binary feature is represented by a Boolean variable, i.e. its value may be 0 (negative) or 1 (positive). An example of such a binary feature is whether the subject is currently suffering from cancer, wherein the value of 0 would answer the question in the negative, and the value of 1 would answer the question in the affirmative. Certain features are continuous features, which means that they can take more than two values. These include height, weight, age, and BMI of a subject. However, in certain embodiments of the present invention, these features may also be represented using a binary variable. For an illustrative but not limiting example, BMI of a subject may be represented as being less than 35 or at least 35, which would correspond to a binary variable value of 0 (negative) and 1 (positive) and could be considered indicative e.g. of obesity status of a subject. In exceptional cases, a feature may also be a discrete variable, for example the blood group of a subject, which may constitute a selection from a finite (but higher than 2) number of elements. Similar to the representation of a continuous feature with a binary feature, continuous feature may also be represented by using a discrete feature. A non-limiting example would be classification of subject to predefined groups according to their age.

As understood herein, preferably within the scope of the present invention, the subject-specific health and life-style data comprise subject physical parameters, course of the past coronavirus disease, data on medication(s) administered to the subject, medical background, and/or life style data. It is deemed that any selection of parameters as encompassed in the list recited hereinabove is encompassed within the scope of the present invention.

Preferably, within the scope of the present invention the subject-specific health and life-style data comprise subject physical parameters. As understood herein, the subject physical parameters as defined hereinabove and within the scope of the present invention preferably comprise weight, height, BMI, blood group, age, and/or sex. It is noted that these parameters are well known to the person skilled in the art and are understood to be represented by the standard definition of each term. Preferably, the subject physical parameters as defined herein comprise age, BMI and sex. Even more preferably, the subject physical parameters as defined herein comprise age. The present inventors have surprisingly found that younger people are more likely to have adverse side effects than the elderly. Without wishing to be bound by the theory, the present inventors postulate it may be due to the differences in the immune systems of the young and the elderly, which in turn cause inconsistencies in response to the vaccine. As understood herein, young people are preferably of not more than 30 years of age. As further understood herein, elderly people are preferably at least 60 years old. In certain preferred embodiments, the subject physical parameters as defined herein comprise sex. Sex is herein preferably defined as biological gender and is represented by a binary variable. The present inventors have surprisingly found that the women are more likely than men to get coronavirus vaccine-related side effects. Without wishing to be bound by the theory, the present inventors postulate that it can be due to a more robust female immune system.

Further preferably within the scope of the present invention the subject-specific health and life-style data comprise course of the past coronavirus disease. As understood herein, within the scope of the present invention the course of the past coronavirus disease comprises data on severity, vertigo, sore throat, headache, chest pain, feeling paralyzed, loss of consciousness, breathing difficulties, loss of smell, digestive difficulties, cough, pain, fatigue and/or fever. Preferably, the course of the past coronavirus disease comprises data on vertigo, fever, digestive difficulties and/or fatigue. More preferably, the course of the past coronavirus disease comprises data on vertigo, fever, and/or digestive difficulties. Even more preferably, the course of the past coronavirus disease comprises data on vertigo, and/or fever. Most preferably,

the course of the past coronavirus disease comprises data on vertigo.

As understood herein severity is preferably assessed by the subject as severe and not severe, it is thus represented by a binary feature. The severe is defined as need for hospitalization and not severe is defined as no need for hospitalization. It is thus preferably represented by a binary feature.

As understood herein, vertigo is preferably assessed by the subject as having or not having a sensation of feeling off balance. It is thus preferably represented by a binary feature.

As understood herein, sore throat is preferably assessed by the subject as having or not having pain, scratchiness, and/or irritation of the throat, any of which often worsens when swallowing. Thus, it is preferably represented by a binary feature.

As understood herein, headache is preferably assessed by the subject as having or not having pain in any region of the head. It is thus preferably represented by a binary feature.

As understood herein, chest pain is preferably assessed by the subject as the presence or the lack of the presence of abnormal pain or discomfort in the chest, preferably between the diaphragm and the base of the neck. Thus, it is preferably represented by a binary feature.

As understood herein, feeling paralyzed is preferably assessed by the subject as the feeling (or the lack thereof) of a loss of strength in and control over a muscle or group of muscles in a part of the body. Thus, it is preferably represented by a binary feature. As understood herein, loss of consciousness is preferably assessed by the subject as the occurrence (or the lack thereof) of a partial or complete loss of consciousness with interruption of awareness of oneself and one's surroundings. Thus, it is preferably represented by a binary feature.

As understood herein, breathing difficulties are preferably assessed by the subject as a subjective experience of breathing discomfort, which as known to the skilled person may consists of qualitatively distinct sensations that vary in intensity, or the lack thereof. Thus, it is preferably represented by a binary feature.

As understood herein, loss of smell is preferably assessed by the subject as the loss of the ability to detect one or more smells, or the lack thereof. Thus, it is preferably represented by a binary feature.

As understood herein, digestive difficulties are preferably assessed by the subject as presence of any health problem that occurs in the digestive tract (or the lack thereof). Thus, it is preferably represented by a binary feature.

As understood herein, cough is preferably assessed by the subject as the condition of expelling air from the lungs suddenly with a sharp, short noise, or the lack thereof. Thus, it is preferably represented by a binary feature.

As understood herein, pain is preferably assessed by the subject as the presence of unpleasant physical sensation caused by illness or injury, or the lack thereof. Thus, it is preferably represented by a binary feature.

As understood herein, fatigue is preferably assessed by the subject by the presence of an overall feeling of tiredness or lack of energy. Thus, it is preferably represented by a binary feature.

As understood herein, fever is preferably assessed by the subject as occurrence of an increase in body temperature, often due to an illness, preferably above 38 °C. Thus, it is preferably represented by a binary feature.

Preferably, the subject-specific health and life-style data comprise data on medication(s) administered to the subject. As understood herein, within the scope of the present invention the data on medication(s) administered to the subject comprise data on immunosuppressive therapy, chemotherapy, steroids (preferably cortone), respiratory spray, and/or hormone drugs. Preferably the data on medication(s) administered to the subject comprise data on immunosuppressive therapy. In certain embodiments of the invention however, the subject-specific health and life-style data does not comprise data on medication(s) administered to the subject.

Immunosuppressive therapy as understood herein is administration of a drug or substance that lowers the activity of the body's immune system. Lowering the activity of the body's immune system is for example necessary for patients that have undergone organ transplant in order to prevent rejection of the transplant by the patient's own immune system. Lowering the activity of the body immune system may also be necessary for the treatment of certain conditions, including autoimmune conditions, for example lupus erythematosus. Non-limiting examples of medications used for immunosuppressive therapy include azathioprine, mycophenolate mofetil, and cyclosporine.

Chemotherapy as preferably understood herein is administration of a drug or substance (which also may be referred to as chemotherapeutic) for the treatment of cancer, preferably by destroying the cancer cells. Typically, chemotherapy works by preventing the cancer cells from dividing. Typical chemotherapeutics include alkylatic agents, antimetabolites, anti-microtubule agents, topoisomerase inhibitors and cytotoxic antibiotics.

Therapy with steroids as defined herein is preferably broadly defined as administration of drugs that comprise the carbon skeleton of gonane, as shown in the formula:

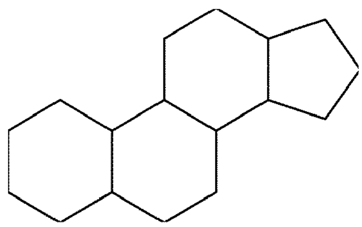

Examples of steroids include estradiol, testosterone and dexamethasone. Respiratory spray as understood herein preferably relates to administration of a drug or substance in a form of aerosol by the means of a respiratory spray. For example, anti-asthma medications are administered in such a way.

Hormone drugs as referred to herein preferably refer to pregnancy or contraception drugs for females that contain hormonal substances such as estrogen and progestin. Preferably, the subject-specific health and life-style data comprise medical background of a subject. As understood herein, within the scope of the present invention the medical background comprises the data on pregnancy, allergy, psychological issues, skeletal system, liver diseases, kidney diseases, digestive tract diseases, blood diseases, immune system diseases, lung diseases, neurological diseases, active cancer diseases, past history of cancer disease, hypertension, heart disease, and/or diabetes. Preferably, the medical background comprises the data on digestive tract diseases, past history of cancer disease and/or allergy. More preferably, the medical background comprises the data on allergy and/or past history of cancer disease. Even more preferably, the medical background comprises the data on allergy.

As defined herein, the terms pregnancy, allergy, psychological issues, skeletal system diseases, liver diseases, kidney diseases, digestive tract diseases, blood diseases, immune system diseases, lung diseases, neurological diseases, active cancer diseases, past history of cancer disease, hypertension, heart disease, and/or diabetes are understandable to the person skilled in the art. As further understood herein, unless explicitly stated otherwise, for the chronic conditions recited herein the medical background with respect to any of these conditions preferably refers to having ever been diagnosed with a said condition. In particular, this applies to allergy, psychological issues, skeletal system diseases, liver diseases, lung diseases, neurological diseases, hypertension, heart diseases and/or diabetes. By the way of an example, the subject with a medical background of allergy, psychological issues, skeletal system diseases, liver diseases, lung diseases, neurological diseases, hypertension, heart diseases and/or diabetes, is a subject previously diagnosed with allergy, psychological issues, skeletal system diseases, liver diseases, lung diseases, neurological diseases, hypertension, heart diseases and/or diabetes, respectively. For certain conditions, as explicitly stated herein, a subject with medical background of said conditions is a subject that is currently characterized by the said conditions. This applies to pregnancy and active cancer disease. Thus, by the way of an example a subject with a medical background of pregnancy is currently pregnant, and a subject with a medical background of an active cancer disease has currently cancer. The medical background of previous cancer disease relates to a subject that had had a cancer disease before but cannot be characterized as a subject with medical background of an active cancer disease. As further understood herein, the features discussed hereinabove are preferably to be represented by binary features within the scope of the methods of the present invention.

As understood herein, psychological issues preferably may include bipolar disorder, depression, psychosis, and schizophrenia.

As understood herein, skeletal system diseases preferably may include arthritis, gout, osteoporosis, Paget's disease, and tendonitis.

As understood herein, liver diseases preferably may include fatty liver, gallstone, hepatitis, liver cirrhosis, and liver failure.

As understood herein, kidney diseases preferably may include chronic kidney disorder, glomerulonephritis, and polycystic kidney disease.

As understood herein, digestive tract diseases preferably may include inflammatory bowel disorders, intestinal polyps, irritable bowel syndrome, malabsorption disorders, stomach reflex, and ulcers.

As understood herein, blood diseases preferably may include anemia, coagulation disorders, favism, alpha thalassemia, and beta thalassemia.

As understood herein, immune system diseases preferably may include AIDS, lupus, and vasculitis.

As understood herein, lung diseases preferably may include asthma, chronic obstructive pulmonary disease, idiopathic pulmonary fibrosis, and pulmonary tuberculosis.

As understood herein, neurological diseases preferably may include Alzheimer's disease, cerebral palsy, dementia, epilepsy, multiple sclerosis, Parkinson's disease, and stroke.

As understood herein, heart diseases preferably may include coronary artery disease, heart valve disorder, hypertrophic cardiomyopathy, heart ischemia, myocarditis, and cardiac rheumatism.

Preferably, the subject specific health and life-style data comprise the life-style data. As understood herein, within the scope of the present invention the life-style data comprise the data on alcohol consumption, use of narcotics, and/or smoking. In certain embodiments of the present invention, the subject specific health and life-style data does not comprise the life-style data as defined herein. Preferably, the life-style data is to be represented as binary feature(s).

Preferably, as encompassed by the present invention, the method for predicting side effects of a coronavirus vaccine in a subject relates to an embodiment, wherein the subject-specific health and life-style data further comprise data on side effects of a previous dose of a coronavirus vaccine. Preferably, the data on side effects of a previous dose of a coronavirus vaccine can be taken into account when the subject has been previously administered with at least one dose of any coronavirus vaccine. It is further understood herein that the previous dose of a coronavirus vaccine may refer to the same coronavirus vaccine that the method of the present invention predicts the side effect thereof, or it may refer to a different coronavirus vaccine. The method of the present invention for predicting side effects of a coronavirus vaccine in a subject as described herein wherein the subject-specific health and life-style data further comprise data on side effects of a previous dose of a coronavirus vaccine is also applicable to subjects that have not received any dose of a coronavirus vaccine.

As understood herein the data on side effects of a previous dose of a coronavirus vaccine preferably encompass the data on side effects of the previous dose of a coronavirus vaccine comprise data on muscle pain, join paint, chills, nausea, headache, fatigue, fever and/or local side effects. As understood herein, the local side effects refer to pain at the injection site, redness at the injection site, and/or swelling at the injection site. As known to the skilled person, side effects, in particular adverse side effects, are one of the main concerns for any vaccine following its administration to a subject. Adverse side effects of different vaccines can range from mild instances to those that may require hospitalization. Adverse side effects may include pain, redness, and swelling at the injection site, joint pain, muscle aches, headache, fatigue, nausea, fever, chills. Other more advanced complications may occur, such as forming blood clots and even anaphylactic shock.

As understood herein the terms muscle pain, joint pain, chills, nausea, headache, fatigue, and/or fever are known to the skilled person and are preferably reported by the subject, as described herein. As understood herein, the subject is preferably to report in the affirmative or in the negative if any of the said adverse side effects as defined hereinabove has occurred to him/her, preferably by using a questionnaire.

As understood herein, the local side effects preferably refer to pain at the injection site, redness at the injection site, and/or swelling at the injection site. They are preferably reported by the subject, as described hereinabove.

Preferably, the method for predicting side effects of a coronavirus vaccine in a subject relates to an embodiment, wherein the previously trained mathematical model is a machine learning model. Preferably, the machine learning model is selected from Logistic Regression (LR), Random Forest (RF), Multi-Layer Perceptron (MLP), K-Nearest Neighbors (KNN), Support Vector Machine (SVM), and Gradient Boosted Decision Trees (XGBoost). More preferably, the machine learning model is Logistic Regression (LR).

As understood herein, the Logistic Regression (LR) is a classification model, a statistical method for binary classification that can be generalized to multiclass classification. As known to the skilled person, Scikit-learn has a highly optimized version of logistic regression implementation, which supports multiclass classification tasks. (Practical Machine Learning for Data Analysis Using Python. https://www.sciencedirect.com/book/9780128213797/practical-machine-learning-for-data-analysis-using-python)

As understood herein, Random Forest (RF) is a supervised learning algorithm. The "forest" it builds, is an ensemble of decision trees, usually trained with the "bagging" method. The general idea of the bagging method is that a combination of learning models increases the overall result. Random forest builds multiple decision trees and merges them together to get a more accurate and stable prediction. (A Complete Guide to the Random Forest Algorithm. https://builtin.com/data-science/random-forest-algorithm)

As understood, herein, a multilayer perceptron (MLP) is a feedforward artificial neural network that generates a set of outputs from a set of inputs. An MLP is characterized by several layers of input nodes connected as a directed graph between the input and output layers. MLP uses backpropagation for training the network. MLP is a deep learning method. (Techopedia. Multilayer Perceptron (MLP). http://www.techopedia.com/definition/20879/multilayer-perceptron-mlp (2017))

As understood herein, K-Nearest Neighbors (kNN) relies only on the most basic assumption underlying all predictions: that observations with similar characteristics will tend to have similar outcomes. Nearest Neighbor methods assign a predicted value to a new observation based on the plurality or mean (sometimes weighted) of its k "Nearest Neighbors" in the training set. Given an infinite amount of data, any observation will have many "neighbors" that are arbitrarily near with respect to all measured characteristics, and the variability of their outcomes will provide as precise a prediction as is theoretically possible barring a perfectly and completely specified model. (K Nearest Neighbor. http://dx.doi.org/10.1016/B978-0-12-801966-5.00009-3 doi:10.1016/B978-0-12-801966-5.00009-3)

As understood herein, Support Vector Machine" (SVM) is a supervised machine learning algorithm that can be used for both classification or regression challenges. However, it is mostly used in classification problems. In the SVM

algorithm, each data item is plotted as a point in n-dimensional space (where n is a number of features) with the value of each feature being the value of a particular coordinate. Then, classification by finding the hyper-plane that differentiates the two classes very well is performed. (Ray, S. Understanding Support Vector Machine (SVM) algorithm from examples (along with code). https://www.analyticsvidhya.com/blog/2017/09/understaing-support-vector-machine-example-code/ (2017))

As understood herein, Gradient-boosted decision trees are a machine learning technique for optimizing the predictive value of a model through successive steps in the learning process. Each iteration of the decision tree involves adjusting the values of the coefficients, weights, or biases applied to each of the input variables being used to predict the target value, with the goal of minimizing the loss function (the measure of the difference between the predicted and actual target values). The gradient is the incremental adjustment made in each step of the process; boosting is a method of accelerating the improvement in predictive accuracy to a sufficiently optimum value. (Gradient-Boosted Decision Trees (GBDT), https://c3.ai/glossary/data-science/gradient-boosted-decision-trees-gbdt/)

In certain embodiments of the present invention, the previously trained mathematical model has been trained using hyperparameter tuning. As known to the skilled person, the hyperparameter tuning is a process in machine learning, wherein the hyperparameter, a parameter that is used to control the learning process, is used. At least one parameter is not optimized in the learning process but is set so that the machine learning model can optimally solve the machine learning problem. Within the method of the present invention, preferably one or more hyperparameter(s) is used. To this end, the present inventors have run a separate hyperparameter tuning job for each machine learning model as disclosed hereinabove and for each side effect to be predicted. This can be accomplished for example by using GridSearchCV or, in particular for higher numbers of hyperparameters to tune, RandomSearch CV, with a Stratified-Cross-Validation. The best model configuration was selected by the present inventors by using the mean ROC-AUC value for the validation set.

In certain embodiments of the present invention, the method for predicting side effects of a coronavirus vaccine in a subject further comprises the step (a') of training of the previously trained mathematical model. The previously trained mathematical model is so trained so that it relies on the predictors. The term predictor is known to the skilled person and may also be referred to as an independent variable. Herein, the predictors are preferably selected from the subject-specific health and life-style data, and may comprise the features selected from subject physical parameters, course of the past coronavirus disease, data on medication(s) administered to the subject, medical background, and/or life style data, as referred to herein. Preferably, the predictors are used as an input of a previously trained mathematical model, as discussed hereinbelow. Further preferably, the training of the previously trained mathematical model relies on one or more hyperparameter, as described hereinabove.

As preferably understood herein, the previously trained mathematical model is trained using the database. The said database, as understood herein, has been compiled for subjects that were previously vaccinated with a coronavirus vaccine.

The database preferably comprises subject-specific data on side effects of a coronavirus vaccine in a subject. This data corresponds to the data output which may also be referred to as predicted side effects and is as discussed hereinbelow. In a preferred embodiment, the subject-specific data on side effects of a coronavirus vaccine in a subject are selected from fever, fatigue, headache, nausea, chills, joint pain, muscle pain and local side effects. As understood herein, the local side effects refer to pain at the injection site, redness at the injection site, and/or swelling at the injection site.

The said database further comprises subject-specific health and life-style data. The said subject-specific health and life-style data is herein used as input of the previously trained mathematical model.

Preferably as understood herein, the subject-specific health and life-style data used as an input of the previously trained mathematical model comprise subject physical parameters, course of the past coronavirus disease, data on medication(s) administered to the subject, medical background, and/or life style data.

As understood herein, the subject physical parameters as defined hereinabove and within the scope of the present invention preferably comprise weight, height, BMI, blood group, age, and/or sex. Preferably, the subject physical parameters as defined herein comprise age, BMI and sex. Even more preferably, the subject physical parameters as defined herein comprise age.

As understood herein, the subject-specific health and life-style data used as an input of a previously trained mathematical model comprise preferably the course of the past coronavirus disease. As understood herein, within the scope of the present invention the course of the past coronavirus disease comprises data on severity, vertigo, sore throat, headache, chest pain, feeling paralyzed, loss of consciousness, breathing difficulties, loss of smell, digestive difficulties, cough, pain, fatigue and/or fever. Preferably, the course of the past coronavirus disease comprises data on vertigo, fever, digestive difficulties and/or fatigue. More preferably, the course of the past coronavirus disease comprises data on vertigo, fever, and/or digestive difficulties. Even more preferably, the course of the past coronavirus disease comprises data on vertigo, and/or fever. Most preferably, the course of the past coronavirus disease comprises data on vertigo.

As understood herein, the subject-specific health and life-style data used as an input of a previously trained mathematical model comprise preferably data on medication(s) administered to the subject. As understood herein, the data on medication(s) administered to the subject comprise data on immunosuppressive therapy, chemotherapy, steroids (preferably cortone), respiratory spray, and/or hormone drugs. Preferably the data on medication(s) administered to the subject comprise data on immunosuppressive therapy. In certain embodiments of the invention however, the subject-specific health and life-style data does not comprise data on medication(s) administered to the subject.

As understood herein, the subject-specific health and life-style data used as an input of a previously trained mathematical model comprise preferably medical background of a subject. As understood herein, the medical background comprises the data on pregnancy, allergy, psychological issues, skeletal system, liver diseases, kidney diseases, digestive tract diseases, blood diseases, immune system diseases, lung diseases, neurological diseases, active cancer diseases, past history of cancer disease, hypertension, heart disease, and/or diabetes. Preferably, the medical background comprises the data on digestive tract diseases, past history of cancer disease and/or allergy. More preferably, the medical background comprises the data on allergy and/or past history of cancer disease. Even more preferably, the medical background comprises the data on allergy.

As understood herein, the subject specific health and life-style data used as an input of a previously trained mathematical model comprise preferably the life-style data. As understood herein, the life-style data comprise the data on alcohol consumption, use of narcotics, and/or smoking. In certain embodiments of the present invention, the subject specific health and life-style data used as an input of a previously trained mathematical model does not comprise the life-style data as defined herein.

As understood herein, the subject-specific health and life-style data used as an input of a previously trained mathematical model may further comprise data on side effects of a previous dose of a coronavirus vaccine. As understood herein the data on side effects of a previous dose of a coronavirus vaccine preferably encompass the data on side effects of the previous dose of a coronavirus vaccine comprise data on muscle pain, join paint, chills, nausea, headache, fatigue, fever and/or local side effects. As understood herein, the local side effects refer to pain at the injection site, redness at the injection site, and/or swelling at the injection site.

As understood herein, certain parameters of the said subject-specific health and life-style data used as an input of a previously trained mathematical model are preferably used by the previously trained mathematical model as predictors. As further understood herein, certain parameters of the said subject-specific health and life-style data used as an input of a previously trained mathematical model are used to train the previously trained mathematical model.

Figure 1 illustrates the flow diagram of the incorporation of data for designing and improving the previously trained mathematical model (herein preferably a machine learning model) and statistical analysis techniques. The training relies on the data of previously vaccinated recipients (100). The input of the system (104) in particular includes data of the dose and type of the vaccine administrated (101) and subject specific health and life-style data (102) for each individual. The said input (104) is subjected to the preanalysis of the input data (105) to make it suitable for the training of the previously trained mathematical model (106). For example, this step (105) may include translating obtained data into binary or discrete features. The data of previously vaccinated recipients (100) is further use to provide the training of the previously trained mathematical model (106) with the data of the side effects that previously occurred (103) for the training purposes. As a result of training, an output is provided which may also be referred to as a finalized system that can predict the possibility of outcomes solely based on the input data.

In certain embodiments of the present invention, the method of the present invention may be implemented as a previously trained mathematical model that is available for use by an end user. Figure 2 illustrates the flow diagram of the previously trained mathematical model (preferably herein machine learning model) and statistical analysis technique for end users. Herein, the end user (for example, a subject that may be eligible for vaccination with a Coronavirus vaccine) provides a system input, which may include subject specific health data (201), subject specific life-style data (202) and data on the side effects of any previous coronavirus vaccine administered to the said subject (203). The system performs then analysis of the input data by using the previously trained mathematical model (204) and predicts the possible adverse side effects of the vaccines (205), according to the input (201-203) of the end user (200). Finally, the results of the said predictions are provided (206). In certain embodiments of the present invention, the said results (206) can be provided in a form of a personalized fact sheet.

Thus, the present invention relates to the method for predicting side effects of a coronavirus vaccine in a subject, wherein the predicted side effects of a coronavirus vaccine are provided in a form of a subject-specific factsheet. The methods of the present invention may include in certain embodiments generating reports, such as a personalized fact sheet that can be provided to vaccine recipients to handle the concerns of vaccines' safety

Preferably, within the scope of the present invention the method for predicting side effects of a coronavirus vaccine in a subject predicts side effects that are selected from fever, fatigue, headache, nausea, chills, joint pain, muscle pain and local side effects. In certain embodiments, the said predicted side effects include fever, fatigue, headache, nausea, chills, joint pain, muscle pain and/or local side effects. It is noted that in certain embodiments of the present invention the predicted side effects may differ depending on whether the prediction is performed for a first vaccination

or any subsequent vaccination. As understood herein, the predicted side effects may be provided as binary features for each side effect, that is, whether the side effect is likely to occur (1) or unlikely to occur (0). In however preferred embodiments of the present invention, the said predicted side effects may be provided in a form of probability of an occurrence of such a side effect, that is in the form of a continuous variable in a range from 0 to 1.

Thus, in certain embodiments of the present invention a personalized fact sheet includes preferably the predicted side effects (e.g. with a calculated possibility between 0 and 1 for each adverse side effect for each subject). Figure 3 illustrates an example of a personalized fact sheet, which is one of the applications of the invention. Figure 3 provides a possibility for each predicted adverse side effect between 0 and 1. It may also consist of other popular and typical parts of fact sheet, such as a brief description of the administrated vaccine.

In a further embodiment, the present invention relates to a method for selecting a subject qualifying for vaccination with a coronavirus vaccine. The method comprises the step (a) of predicting the side effects of a coronavirus vaccine in a subject using the method for predicting the side effects of a coronavirus vaccine in a subject as disclosed herein. The method for selecting a subject qualifying for vaccination with a coronavirus vaccine further comprises the step (b) of selecting a subject qualifying for vaccination with a coronavirus vaccine based on predicted side effects of a coronavirus vaccine in said subject.

In certain embodiments of the present invention, the step (a) of the method for selecting a subject qualifying for vaccination with a coronavirus vaccine can be performed for different coronavirus vaccines. Comparison of predicted side effects for different vaccines may constitute the basis for the step (b) of the said method for selecting a subject qualifying for vaccination with a coronavirus vaccine. In certain embodiments of the present invention, the method for selecting a subject qualifying for vaccination with a coronavirus vaccine may comprise obtaining personalized factsheets for a subject for different coronavirus vaccines in step (a) of the method for selecting a subject qualifying for vaccination with a coronavirus vaccine, and comparing them with each other in order to inform the step (b) of the method for selecting a subject qualifying for vaccination with a coronavirus vaccine.

As understood herein, the method for predicting the side effects of a coronavirus vaccine in a subject of the present invention according to the embodiments as disclosed herein and/or the method for selecting a subject qualifying for vaccination with a coronavirus vaccine of the present invention according to the embodiments as disclosed herein is/are not limited with regard to any particular coronavirus vaccine. The coronavirus vaccine may be selected from DNA vaccine, RNA vaccine, adenovirus vector vaccine, inactivated virus vaccine, and subunit vaccine.

As referred to herein, the term AstraZeneca vaccine preferably refers to a Covid-19 vaccine known as Vaxzevria. As referred to herein, the term Sputnik V vaccine preferably refers to a Covid-19 vaccine also known as Gam-COVID-Vac.

Within the scope of the present invention, in particular within the scope of the method for predicting the side effects of a coronavirus vaccine in a subject of the present invention according to the embodiments as disclosed herein and/or the method for selecting a subject qualifying for vaccination with a coronavirus vaccine of the present invention according to the embodiments as disclosed herein the coronavirus is preferably SARS-CoV2. Thus, the coronavirus vaccine is preferably a vaccine against SARS-CoV2 coronavirus, in other words the coronavirus vaccine is preferably a Covid-19 vaccine.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

**References**

1. WHO Director-General's opening remarks at the media briefing on COVID-19 - 11 March 2020. https://www.who.int/director-general/speeches/detail/who-director-general-s-opening-remarks-at-the-media-briefing-on-covid-19---11-march-2020. Accessed 17 May 2021

2. (2021) No winners but fewer losers in global economy from COVID than expected. https://news.un.org/en/story/2021/03/1087712. Accessed 17 May 2021

3. Nejadghaderi SA, Saghazadeh A, Rezaei N (2021) Health Care Policies and COVID-19 Prevalence: Is There Any Association? Int J Health Serv 20731421993940

4. Diagnostics laboratory emergency use listing. https://www.who.int/teams/regulation-prequalification/eul. Accessed 17 May 2021

5. CDC COVID-19 Response Team, Food and Drug Administration (2021) Allergic Reactions Including Anaphylaxis After Receipt of the First Dose of Moderna COVID-19 Vaccine - United States, December 21, 2020-January 10, 2021. MMWR Morb Mortal Wkly Rep 70:125-129

6. Riad A, Pokorná A, Attia S, Klugarová J, Koščík M, Klugar M (2021) Prevalence of COVID-19 Vaccine Side Effects among Healthcare Workers in the Czech Republic. J Clin Med Res. https://doi.org/10.3390/jcm10071428
7. Klein SL, Marriott I, Fish EN (2015) Sex-based differences in immune function and responses to vaccination. Trans R Soc Trop Med Hyg 109:9-15
8. Kopsaftis Z, Wood-Baker R, Poole P (2018) Influenza vaccine for chronic obstructive pulmonary disease (COPD). Cochrane Database Syst Rev 6:CD002733
9. Fekrvand S, Yazdani R, Olbrich P, et al (2020) Primary Immunodeficiency Diseases and Bacillus Calmette-Guérin (BCG)-Vaccine-Derived Complications: A Systematic Review. J Allergy Clin Immunol Pract 8:1371-1386

Further aspects and/or embodiments of the invention are disclosed in the following numbered items.

1. A method for predicting side effects of a coronavirus vaccine in a subject, the method comprising:

(a) providing subject-specific health and life-style data; and
(b) predicting the side effects of a coronavirus vaccine in a subject based on the subject-specific health and life-style data using a previously trained mathematical model.

2. The method of item 1, wherein the subject-specific health and life-style data comprise subject physical parameters, course of the past coronavirus disease, data on medication(s) administered to the subject, medical background, and/or life style data, preferably wherein the subject-specific health and life-style data is collected by using a questionnaire.
3. The method of item 2, wherein the subject physical parameters comprise weight, height, BMI, blood group, age, and/or sex.
4. The method of any one of items 2 to 3, wherein the course of the past coronavirus disease comprises data on severity, vertigo, sore throat, headache, chest pain, feeling paralyzed, loss of consciousness, breathing difficulties, loss of smell, digestive difficulties, cough, pain, fatigue and/or fever.
5. The method of any one of items 2 to 4, wherein the data on medication(s) administered to the subject comprise data on immunosuppressive therapy, chemotherapy, steroids (preferably cortone), respiratory spray, and/or hormone drugs.
6. The method of any one of items 2 to 5, wherein the medical background comprises the data on pregnancy, allergy, psychological issues, skeletal system, liver diseases, kidney diseases, digestive tract diseases, blood diseases, immune system diseases, lung diseases, neurological diseases, active cancer diseases, past history of cancer disease, hypertension, heart disease, and/or diabetes.
7. The method of any one of items 2 to 6, wherein the life-style data comprise the data on alcohol consumption, use of narcotics, and/or smoking.
8. The method of any one of items 2 to 7, wherein the subject-specific health and life-style data further comprise data on side effects of a previous dose of a coronavirus vaccine.
9. The method of item 8, wherein the data on side effects of the previous dose of a coronavirus vaccine comprise data on muscle pain, join paint, chills, nausea, headache, fatigue, fever and/or local side effects.
10. The method of any one of items 1 to 9, wherein the previously trained mathematical model is a machine learning model.
11. The method of item 10, wherein the machine learning model is selected from Logistic Regression (LR), Random Forest (RF), Multi-Layer Perceptron (MLP), K-Nearest Neighbors (KNN), Support Vector Machine (SVM), and Gradient Boosted Decision Trees (XGBoost).
12. The method of item 11, wherein the machine learning model is Logistic Regression (LR).
13. The method of any one of items 10 to 12, wherein the previously trained mathematical model has been trained using hyperparameter tuning.
14. The method of any one of items 1 to 13, wherein the predicted side effects of administering a vaccine to a subject include fever, fatigue, headache, nausea, chills, joint pain, muscle pain and/or local side effects.
15. The method of any one of items 1 to 14, wherein the method further comprises the step of (a') training of the previously trained mathematical model.
16. The method of any one of items 1 to 15, wherein the previously trained mathematical model is trained using the database comprising subject-specific data on side effects of a coronavirus vaccine in a subject and subject-specific health and life-style data, wherein the database has been compiled for subjects that were previously vaccinated with a coronavirus vaccine.
17. The method of item 16, wherein the data on side effects of a coronavirus vaccine in a subject is as described in item 14,
18. The method of item 16 or 17, wherein the subject-specific health and life-style data are as described in any one

of items 2 to 9.

19. The method of any one of items 1 to 18, wherein the previously trained mathematical model relies on predictors selected from subject-specific health and life-style data, wherein the subject specific health and life-style data are as described in any one of items 2 to 9.

20. The method of any one of items 1 to 19, wherein the coronavirus vaccine is selected from DNA vaccine, RNA vaccine, adenovirus vector vaccine, inactivated virus vaccine, and subunit vaccine.

21. The method of any one of items 1 to 20, wherein the predicted side effects of a coronavirus vaccine are provided in a form of a subject-specific factsheet.

22. A method for selecting a subject qualifying for vaccination with a coronavirus vaccine, the method comprising

(a) predicting the side effects of a coronavirus vaccine in a subject using the method according to any one of items 1 to 21, and

(b) selecting a subject qualifying for vaccination with a coronavirus vaccine based on predicted side effects of a coronavirus vaccine in said subject.

23. The method of any one of preceding items, wherein the coronavirus is SARS-CoV2.

## Examples

The following examples are merely illustrative of the present invention and should not be construed to limit the scope of the invention which is defined by the appended claims.

*Introduction*

The models to predict adverse side effects following the first and second doses of the Sputnik V vaccine and the first dose of AstraZeneca vaccine were created. The Side effects as the output data included eight parameters: fever, fatigue, headache, nausea, chills, joint pain, muscle pain, and local side effects. The prediction model for the first dose was trained using data from 3740 patients receiving the Sputnik V vaccine and 2537 patients receiving the AstraZeneca vaccine.

The first dose prediction models were trained on age, gender, BMI, lifestyle variables, history and severity of past COVID-19 infection and its symptoms, and medical background of a subject. The total number of input parameters for this model was 46 features. The second dose prediction for Sputnik V was trained on the data from 2284 people who got both the first and second vaccine dose. For the prediction model of the adverse effects following the second dose of Sputnik V vaccine we included data from 2284 patients who had received both doses of the vaccine. For the second dose prediction of Sputnik V the adverse side effects of the first dose of vaccination were also included as the input data, which made the total number of input parameters for the second prediction model 54 features.

*Data*

In this cohort study, an online survey was used to gather adequate data to develop the models. The relevant healthcare authorities informed the vaccination recipients at the injection site to fill the online survey three days after vaccination. All the participants in the study were healthcare personnel at 90 different hospitals in Tehran, Iran. There were no specific inclusion and exclusion criteria in our research outside the criteria used for vaccine eligibility in the first place. The online survey was available from April 10, 2021, and is still available to the vaccinated Recipients (as of September 16, 2021). The final database used to design the models was obtained by aggregating the data of 3740 vaccine recipients that had completed the online survey before May 10, 2021 for the Sputnik V vaccine and 2537 vaccine recipients that had received the first shot of AstraZeneca vaccine. Of these 3740 persons from the Sputnik V group, all of them had the first dose of the vaccine, and 2288 had the second dose of Sputnik V as well. Due to the long waiting between the first and second dose of AstraZeneca vaccine the model is limited herein to the first dose of this vaccine.

The study was approved by the Iran University of Medical Sciences Ethics Committee.

*Predictors*

The patients' age, sex, blood group type, smoking habit, drug abuse, and alcohol dependency status, BMI index, past medical history (PMH), including their comorbidities or use of specific drugs and certain conditions, and finally, their COVID-19 history (were they infected or not, with what severity and what kind of symptoms they had), were used as the predictors for the models of first dose of Sputnik V and AstraZeneca vaccine. The prediction model for the second dose of Sputnik V also has access to vaccine receivers' first dose side effects data. The total number of predictors

for the first and second dose models is 46 and 54, respectively. The selection of variables as predictors was based on the available recorded data survey. All these predictors were recorded via an online form explicitly filled by the healthcare personnel three days or more after their vaccination.

Most input parameters and all output parameters were encoded as binary variables using One-hot encoding. One-hot encoding, as known to the skilled person, is a method of creating binary parameters for and based on categorical parameters, indicating the presence of each possible value from the original data with 1 and the absence with 0. Continuous predictors including age and BMI were normalized using a MinMax scaler. To avoid feeding models with outlier values that may have been entered due to the unintentional mistakes while completing the form, any record that contains outlier values for age (<18 years and >120 years), height (<100 cm and >200 cm), and weight (<30 kg and >200 kg) was excluded.

*Machine learning methods* as *previously trained mathematical model*

To ensure that models would not be overfitted on training data and will be generalizable to unseen real-world data, 20% of the data was kept unseen from the model, these data may also be referred to as a test dataset. On the other 80%, a 5-fold cross-validation algorithm was performed. For this purpose, all of the records were split into five subsets at random. Four subsets were used as training data, and one subset was held back for model testing as a validation set. The cross-validation process was repeated four times more, with each of the five subsets being used exactly once as the validation data. Afterward, model performance metrics were calculated separately for each model training and validation group.

To compare training and validation metrics, they need to have almost the same proportion from positive and negative data points. This can be achieved by splitting vaccine receivers who showed side effects from those who did not show that specific side effect and putting them into five stratified subsets and then combining them into the final five subsets. This way, the same proportion of positive and negative persons for the side effect in each of the final five subsets was maintained.

Several machine learning techniques for both models were evaluated: Logistic Regression (LR), Random Forest (RF), Multi-Layer Perceptron (MLP), K-Nearest Neighbors (KNN), Support Vector Machine (SVM), and Gradient Boosted Decision Trees (XGBoost).

Scikit-learn machine learning library was used to implement both preprocessing algorithms and models (Garreta, R., & Moncecchi, G. (2013). Learning scikit-learn: Machine Learning in Python. Packt Publishing) and the xgboost package was used for training Gradient Boosted Decision Trees ( http://doi.acm.org/10.1145/2939672.2939785.).

For all three models of doses of vaccines and each side effect group, models' performance in 5-fold cross-validation has been evaluated using accuracy, ROC-AUC, precision, and recall.

To achieve the best possible performance for each model on the prediction of each side effect, separate hyperparameter tuning job was run on each model and each target side effect. GridSearchCV (RandomSearchCV for models with more parameters to tune) with a Stratified-Cross-Validation was used to this end. Finally, the best model configuration has been selected using the mean ROC-AUC value for the validation set. It is notable that as in each iteration of hyperparameter tuning, the improvement of the performance metric on the validation dataset is aimed for, which can lead to overfitting the model on validation data. To ensure that our model is not overfitted, the metrics from training, validation, and unseen test sets are compared with each other.

The frequency of each side effect in our dataset has been shown in Figure 4A for both the first doses of AstraZeneca and Sputnik V vaccine and the second dose of Sputnik V. For all three models, local side effects in the injection site were the most frequent (65% and 62% for first and second doses of Sputnik V vaccine, respectively and 77% for the first dose of AstraZeneca vaccine), and nausea was the least frequent side effect (10% for both doses of Sputnik V and 21% for the first dose of AstraZeneca vaccine ). Figure 4B, and Figure 4C shows the distribution of some of these features, including sex, smoking, covid-19 prior infection, age, and BMI.. Sputnik V vaccine feature distributions are for 3740 persons that got at least one vaccine dose; the second dose model uses a subset of these data that also got their second dose. Astrazeneca vaccine feature distributions are for 2537 persons that got at least one vaccine dose.

*Previously trained mathematical model*

Six machine learning methods were evaluated for all three groups of vaccine injections, which are listed, together with the hyperparameters used in Table 1 for the first dose of AstraZeneca vaccine, Table 2 for the first dose and Table 3 for the second dose of Sputnik V vaccine. The best parameters for each of the six models for all three groups has been calculated by the hyperparameter tuning using Cross-Validation (test dataset kept unseen in this step).

Table 1. Tuned hyperparameters as used in the prediction of side effects for the first dose of AstraZeneca vaccine. As it is to be understood herein, Dose1_Fever, Dose1_Fatigue, Dose1_Headache, Dose1_Nausea, Dose1_Chills, Dose1_Joint_Pain, Dose1_Muscle_Pain, Dose1_Local refer to the predicted side effects for the first dose

of the AstraZeneca vaccine, namely fever, fatigue, headache, nausea, chills, joint pain, muscle pain and local side effects, respectively. For the Logistic Regression mode, C is herein understood as inverse of regularization strength and is preferably provided as a positive float number, and solver is an algorithm that performs the regression, herein lbfgs stands for limited-memory Broyden-Fletcher-Goldfarb-Shanno, and liblinear stands for library for large linear classification. For the SVM model, C is herein understood as penalty parameter of the error term, gamma is the parameter that defines how far the influence of a single training example reaches; scale gamma parameter means that [please provide details], and kernel is defined as function used in SVM for helping to solve problems; linear kernel means that [...]. In XGBClassifier model n_estimators relates to the number of decision trees, min_child_weight relates to a minimum sum of instance weight (hessian) needed in a child, i.e. ich the tree partition step results in a leaf node with the sum of instance weight less than min_child_weight, the building process will give up further partitioning; max-depth is the maximum depth that the tree is allowed to grow to, in other words the longest path between the root node and the leaf node; and learning_rate parameter controls the weighting of new trees added to the model. In Random Forest (RF) model, max_depth defines the longest path between the root node and the leaf node; min_samples_split defines minimum number of samples required to split an internal node; and n_estimators is the number of trees that are to be built before taking the maximum voting or averages of predictions. In KNN n_neighbors is the number of the nearest neighbors; and weights defines the function that fives a weight to the nearest points, herein "distance" means that [please provide]. In MLP model, activation refers to the activation function used in the hidden layers, herein tanh means that [please provide]; hidden_layer_sizes refers to [please provide], herein (100,100) means that [please provide]; learning_rate can be constant or adaptive and it is a parameter that [please provide]; solver is an algorithm that performs the regression, herein used sgd stands for Stochastic Gradient Descent. It is noted that the parameters as defined hereinabove and as used in Table 1 are known to the person skilled in the art.

Table 1

| **Side Effect** | **Model Type** | **Tuned hyperparameters** |
|---|---|---|
| Dose1_Fever | Logistic_Regression | C: 1.0, solver: lbfgs |
| Dose1_Fatigue | Logistic_Regression | C: 0.5, solver: lbfgs |
| Dose1_Headache | Logistic_Regression | C: 0.1, solver: liblinear |
| Dose1_Nausea | Logistic_Regression | C: 0.1, solver: liblinear |
| Dose1_Chills | Logistic_Regression | C: 0.5, solver: lbfgs |
| Dose1_Joint_Pain | Logistic_Regression | C: 0.5, solver: liblinear |
| Dose1_Muscle_Pain | Logistic_Regression | C: 0.1, solver: lbfgs |
| Dose1_Local | Logistic_Regression | C: 0.5, solver: lbfgs |
| Dose1_Fever | SVM | C: 10, gamma: scale, kernel: linear |
| Dose1_Fatigue | SVM | C: 1, gamma: scale, kernel: linear |
| Dose1_Headache | SVM | C: 10, gamma: scale, kernel: linear |
| Dose1_Nausea | SVM | C: 10, gamma: scale, kernel: linear |
| Dose1_Chills | SVM | C: 1, gamma: scale, kernel: linear |
| Dose1_Joint_Pain | SVM | C: 1, gamma: scale, kernel: linear |
| Dose1_Muscle_Pain | SVM | C: 10, gamma: scale, kernel: linear |
| Dose1_Local | SVM | C: 0.1, gamma: scale, kernel: linear |
| Dose1_Fever | XGBClassifier | n_estimators: 150, min_child_weight: 3, max_depth: 6, learning_ rate: 0.01 |
| Dose1_Fatigue | XGBClassifier | n_estimators: 50, min_child_weight: 4, max_depth: 8, learning_ rate: 0.1 |
| Dose1_Headache | XGBClassifier | n_estimators: 100, min_child_weight: 2, max_depth: 9, learning_ rate: 0.1 |
| Dose1_Nausea | XGBClassifier | n_estimators: 150, min_child_weight: 3, max_depth: 10, learning_ rate: 0.1 |

(continued)

| Side Effect | Model Type | Tuned hyperparameters |
|---|---|---|
| Dose1_Chills | XGBClassifier | n_estimators: 50, min_child_weight: 1, max_depth: 8, learning_ rate: 0.1 |
| Dose1_Joint_Pain | XGBClassifier | n_estimators: 50, min_child_weight: 3, max_depth: 9, learning_ rate: 0.1 |
| Dose1_Muscle_Pain | XGBClassifier | n_estimators: 50, min_child_weight: 3, max_depth: 10, learning_ rate: 0.1 |
| Dose1_Local | XGBClassifier | n_estimators: 50, min_child_weight: 4, max_depth: 6, learning_ rate: 0.1 |
| Dose1_Fever | RF | max_depth: 9, min_samples_split: 2, n_estimators: 50 |
| Dose1_Fatigue | RF | max_depth: 10, min_samples_split: 3, n_estimators: 150 |
| Dose1_Headache | RF | max_depth: 10, min_samples_split: 2, n_estimators: 150 |
| Dose1_Nausea | RF | max_depth: 10, min_samples_split: 3, n_estimators: 50 |
| Dose1_Chills | RF | max_depth: 10, min_samples_split: 3, n_estimators: 100 |
| Dose1_Joint_Pain | RF | max_depth: 10, min_samples_split: 2, n_estimators: 50 |
| Dose1_Muscle_Pain | RF | max_depth: 10, min_samples_split: 4, n_estimators: 50 |
| Dose1_Local | RF | max_depth: 10, min_samples_split: 4, n_estimators: 100 |
| Dose1_Fever | KNN | n_neighbors: 13, weights: distance |
| Dose1_Fatigue | KNN | n_neighbors: 13, weights: distance |
| Dose1_Headache | KNN | n_neighbors: 13, weights: distance |
| Dose1_Nausea | KNN | n_neighbors: 13, weights: distance |
| Dose1_Chills | KNN | n_neighbors: 13, weights: distance |
| Dose1_Joint_Pain | KNN | n_neighbors: 13, weights: distance |
| Dose1_Muscle_Pain | KNN | n_neighbors: 9, weights: distance |
| Dose1_Local | KNN | n_neighbors: 13, weights: distance |
| Dose1_Fever | MLP | activation: tanh, hidden_layer_sizes: (100, 100), learning_rate: adaptive, solver: sgd |
| Dose1_Fatigue | MLP | activation: tanh, hidden_layer_sizes: (100, 100), learning_rate: constant, solver: sgd |
| Dose1_Headache | MLP | activation: tanh, hidden_layer_sizes: (100, 100), learning_rate: constant, solver: sgd |
| Dose1_Nausea | MLP | activation: tanh, hidden_layer_sizes: (100, 100), learning_rate: adaptive, solver: sgd |
| Dose1_Chills | MLP | activation: tanh, hidden_layer_sizes: (100, 100), learning_rate: constant, solver: sgd |
| Dose1_Joint_Pain | MLP | activation: tanh, hidden_layer_sizes: (100, 100), learning_rate: adaptive, solver: sgd |
| Dose1_Muscle_Pain | MLP | activation: tanh, hidden_layer_sizes: (100, 100), learning_rate: adaptive, solver: sgd |
| Dose1_Local | MLP | activation: tanh, hidden_layer_sizes: (100, 100), learning_rate: constant, solver: sgd |

**Table 2.** Tuned hyperparameters as used in the prediction of side effects for the first dose of Sputnik V vaccine.

As it is to be understood herein, Dose1_Fever, Dose1_Fatigue, Dose1_Headache, Dose1_Nausea, Dose1_Chills, Dose1_Joint_Pain, Dose1_Muscle_Pain, Dose1_Local refer to the predicted side effects for the first dose of the Sputnik V vaccine, namely fever, fatigue, headache, nausea, chills, joint pain, muscle pain and local side effects, respectively. For the description of hyperparameters, see the description of the contents of Table 1 hereinabove.

Table 2

| Side Effect | Model Type | Tuned Hyperparameters |
|---|---|---|
| Dose1_Fever | Logistic_Regression | C: 1.0, solver: lbfgs |
| Dose1_Fatigue | Logistic_Regression | C: 0.5, solver: liblinear |
| Dose1_Headache | Logistic_Regression | C: 0.5, solver: liblinear |
| Dose1_Nausea | Logistic_Regression | C: 0.5, solver: lbfgs |
| Dose1_Chills | Logistic_Regression | C: 10.0, solver: lbfgs |
| Dose1_Joint_Pain | Logistic_Regression | C: 1.0, solver: lbfgs |
| Dose1_Muscle_Pain | Logistic_Regression | C: 1.0, solver: lbfgs |
| Dose1_Local | Logistic_Regression | C: 0.1, solver: lbfgs |
| Dose1_Fever | SVM | C: 1, gamma: scale, kernel: linear |
| Dose1_Fatigue | SVM | C: 1, gamma: scale, kernel: linear |
| Dose1_Headache | SVM | C: 1, gamma: scale, kernel: linear |
| Dose1_Nausea | SVM | C: 0.1, gamma: scale, kernel: linear |
| Dose1_Chills | SVM | C: 1, gamma: scale, kernel: linear |
| Dose1_Joint_Pain | SVM | C: 0.1, gamma: scale, kernel: linear |
| Dose1_Muscle_Pain | SVM | C: 10, gamma: scale, kernel: linear |
| Dose1_Local | SVM | C: 0.1, gamma: scale, kernel: linear |
| Dose1_Fever | XGBClassifier | n_estimators: 50, min_child_weight: 4, max_depth: 6, learning_rate: 0.1 |
| Dose1_Fatigue | XGBClassifier | n_estimators: 150, min_child_weight: 1, max_depth: 8, learning_rate: 0.1 |
| Dose1_Headache | XGBClassifier | n_estimators: 50, min_child_weight: 1, max_depth: 8, learning_rate: 0.1 |
| Dose1_Nausea | XGBClassifier | n_estimators: 50, min_child_weight: 2, max_depth: 9, learning_rate: 0.1 |
| Dose1_Chills | XGBClassifier | n_estimators: 50, min_child_weight: 3, max_depth: 8, learning_rate: 0.1 |
| Dose1_Joint_Pain | XGBClassifier | n_estimators: 50, min_child_weight: 3, max_depth: 6, learning_rate: 0.1 |
| Dose1_Muscle_Pain | XGBClassifier | n_estimators: 100, min_child_weight: 1, max_depth: 10, learning_rate: 0.1 |
| Dose1_Local | XGBClassifier | n_estimators: 50, min_child_weight: 1, max_depth: 6, learning_rate: 0.1 |
| Dose1_Fever | RF | max_depth: 10, min_samples_split: 2, n_estimators: 150 |
| Dose1_Fatigue | RF | max_depth: 10, min_samples_split: 3, n_estimators: 150 |
| Dose1_Headache | RF | max_depth: 10, min_samples_split: 3, n_estimators: 150 |
| Dose1_Nausea | RF | max_depth: 10, min_samples_split: 4, n_estimators: 100 |
| Dose1_Chills | RF | max_depth: 10, min_samples_split: 2, n_estimators: 100 |

(continued)

| Side Effect | Model Type | Tuned Hyperparameters |
|---|---|---|
| Dose1_Joint_Pain | RF | max_depth: 10, min_samples_split: 2, n_estimators: 100 |
| Dose1_Muscle_Pain | RF | max_depth: 10, min_samples_split: 4, n_estimators: 100 |
| Dose1_Local | RF | max_depth: 10, min_samples_split: 2, n_estimators: 150 |
| Dose1_Fever | KNN | n_neighbors: 13, weights: distance |
| Dose1_Fatigue | KNN | n_neighbors: 13, weights: distance |
| Dose1_Headache | KNN | n_neighbors: 13, weights: distance |
| Dose1_Nausea | KNN | n_neighbors: 13, weights: distance |
| Dose1_Chills | KNN | n_neighbors: 13, weights: distance |
| Dose1_Joint_Pain | KNN | n_neighbors: 13, weights: distance |
| Dose1_Muscle_Pain | KNN | n_neighbors: 13, weights: distance |
| Dose1_Local | KNN | n_neighbors: 13, weights: distance |
| Dose1_Fever | MLP | activation: tanh, hidden_layer_sizes: (100, 100), learning_rate: constant, solver: sgd |
| Dose1_Fatigue | MLP | activation: tanh, hidden_layer_sizes: (100, 100), learning_rate: constant, solver: sgd |
| Dose1_Headache | MLP | activation: tanh, hidden_layer_sizes: (100, 100), learning_rate: adaptive, solver: sgd |
| Dose1_Nausea | MLP | activation: tanh, hidden_layer_sizes: (100, 100), learning_rate: constant, solver: sgd |
| Dose1_Chills | MLP | activation: tanh, hidden_layer_sizes: (100, 100), learning_rate: constant, solver: sgd |
| Dose1_Joint_Pain | MLP | activation: tanh, hidden_layer_sizes: (100, 100), learning_rate: constant, solver: sgd |
| Dose1_Muscle_Pain | MLP | activation: tanh, hidden_layer_sizes: (100, 100), learning_rate: adaptive, solver: sgd |
| Dose1_Local | MLP | activation: tanh, hidden_layer_sizes: (100, 100), learning_rate: constant, solver: sgd |

**Table 3.** Tuned hyperparameters as used in the prediction of side effects for the second (last) dose of Sputnik V vaccine. As it is to be understood herein, Dose_Last_Fever, Dose_Last_Fatigue, Dose_Last_Headache, Dose_Last_Nausea, Dose_Last_Chills, Dose_Last_Joint_Pain, Dose_Last_Muscle_Pain, Dose_Last_Local refer to the predicted side effects for the second (last) dose of the Sputnik V vaccine, namely fever, fatigue, headache, nausea, chills, joint pain, muscle pain and local side effects, respectively. For the description of hyperparameters, see the description of the contents of Table 1 hereinabove.

Table 3

| Side Effect | Model Type | Tuned Hyperparameters |
|---|---|---|
| Dose_Last_Fever | Logistic_Regression | C: 1.0, solver: liblinear |
| Dose_Last_ Fatigue | Logistic_Regression | C: 0.1, solver: lbfgs |
| Dose_Last_ Headache | Logistic_Regression | C: 0.1, solver: liblinear |
| Dose_Last_ Nausea | Logistic_Regression | C: 0.1, solver: liblinear |

(continued)

| Side Effect | Model Type | Tuned Hyperparameters |
|---|---|---|
| Dose_Last_Chills | Logistic_Regression | C: 0.1, solver: liblinear |
| Dose_Last_Joint_ Pain | Logistic_Regression | C: 0.1, solver: liblinear |
| Dose_Last_ Muscle_Pain | Logistic_Regression | C: 0.01, solver: Ibfgs |
| Dose_Last_Local | Logistic_Regression | C: 0.1, solver: Ibfgs |
| Dose_Last_Fever | SVM | C: 10, gamma: scale, kernel: linear |
| Dose_Last_ Fatigue | SVM | C: 0.1, gamma: scale, kernel: linear |
| Dose_Last_ Headache | SVM | C: 1, gamma: scale, kernel: linear |
| Dose_Last_ Nausea | SVM | C: 1, gamma: scale, kernel: linear |
| Dose_Last_Chills | SVM | C: 1, gamma: scale, kernel: linear |
| Dose_Last_Joint_ Pain | SVM | C: 0.1, gamma: scale, kernel: linear |
| Dose_Last_ Muscle_Pain | SVM | C: 10, gamma: scale, kernel: linear |
| Dose_Last_Local | SVM | C: 0.1, gamma: scale, kernel: linear |
| Dose_Last_Fever | XGBClassifier | n_estimators: 150, min_child_weight: 4, max_depth: 9, learning_rate: 0.01 |
| Dose_Last_ Fatigue | XGBClassifier | n_estimators: 50, min_child_weight: 3, max_depth: 8, learning_rate: 0.1 |
| Dose_Last_ Headache | XGBClassifier | n_estimators: 100, min_child_weight: 1, max_depth: 6, learning_rate: 0.1 |
| Dose_Last_ Nausea | XGBClassifier | n_estimators: 100, min_child_weight: 2, max_depth: 9, learning_rate: 0.1 |
| Dose_Last_Chills | XGBClassifier | n_estimators: 150, min_child_weight: 1, max_depth: 8, learning_rate: 0.1 |
| Dose_Last_Joint_ Pain | XGBClassifier | n_estimators: 50, min_child_weight: 4, max_depth: 9, learning_rate: 0.1 |
| Dose_Last_ Muscle_Pain | XGBClassifier | n_estimators: 100, min_child_weight: 2, max_depth: 6, learning_rate: 0.1 |
| Dose_Last_Local | XGBClassifier | n_estimators: 100, min_child_weight: 4, max_depth: 6, learning_rate: 0.1 |
| Dose_Last_Fever | RF | max_depth: 10, min_samples_split: 2, n_estimators: 150 |
| Dose_Last_ Fatigue | RF | max_depth: 10, min_samples_split: 2, n_estimators: 150 |
| Dose_Last_ Headache | RF | max_depth: 9, min_samples_split: 2, n_estimators: 100 |
| Dose_Last_ Nausea | RF | max_depth: 10, min_samples_split: 3, n_estimators: 100 |
| Dose_Last_Chills | RF | max_depth: 10, min_samples_split: 2, n_estimators: 150 |

(continued)

| Side Effect | Model Type | Tuned Hyperparameters |
|---|---|---|
| Dose_Last_Joint_ Pain | RF | max_depth: 10, min_samples_split: 2, n_estimators: 100 |
| Dose_Last_ Muscle_Pain | RF | max_depth: 10, min_samples_split: 4, n_estimators: 50 |
| Dose_Last_Local | RF | max_depth: 10, min_samples_split: 2, n_estimators: 150 |
| Dose_Last_Fever | KNN | n_neighbors: 13, weights: distance |
| Dose_Last_ Fatigue | KNN | n_neighbors: 13, weights: distance |
| Dose_Last_ Headache | KNN | n_neighbors: 13, weights: distance |
| Dose_Last_ Nausea | KNN | n_neighbors: 13, weights: distance |
| Dose_Last_Chills | KNN | n_neighbors: 13, weights: distance |
| Dose_Last_Joint_ Pain | KNN | n_neighbors: 13, weights: distance |
| Dose_Last_ Muscle_Pain | KNN | n_neighbors: 13, weights: distance |
| Dose_Last_Local | KNN | n_neighbors: 13, weights: distance |
| Dose_Last_Fever | MLP | activation: relu, hidden_layer_sizes: (100, 100), learning_rate: adaptive, solver: sgd |
| Dose_Last_ Fatigue | MLP | activation: tanh, hidden_layer_sizes: (200,), learning_rate: constant, solver: sgd |
| Dose_Last_ Headache | MLP | activation: relu, hidden_layer_sizes: (200,), learning_rate: constant, solver: sgd |
| Dose_Last_ Nausea | MLP | activation: relu, hidden_layer_sizes: (100,), learning_rate: adaptive, solver: sgd |
| Dose_Last_Chills | MLP | activation: relu, hidden_layer_sizes: (100,), learning_rate: constant, solver: sgd |
| Dose_Last_Joint_ Pain | MLP | activation: relu, hidden_layer_sizes: (200,), learning_rate: constant, solver: sgd |
| Dose_Last_ Muscle_Pain | MLP | activation: relu, hidden_layer_sizes: (100, 100), learning_rate: constant, solver: sgd |
| Dose_Last_Local | MLP | activation: relu, hidden_layer_sizes: (100,), learning_rate: adaptive, solver: sgd |

*Model performance*

*First dose of Sputnik V and AstraZeneca side effect prediction*

Side effect prediction can be considered as 8 separate classification problems. For each of these side effects, all of the 6 model types were trained and evaluated were the average value for accuracy and ROC-AUC, precision and recall for all 5 validation sets and unseen test sets. Parameters of each model on each side effect have been optimized based on validation ROC-AUC value and the best model configuration has been used for the test set prediction. As it has been shown in Figure 5 for the Sputnik V vaccine and Figure 6 for the AstraZeneca vaccine, all of the model types (except KNN) have shown comparable performance on validation sets, but these models differ in their training set values.

As known to the skilled person, the receiver operating characteristic (ROC) curve is commonly used to assess

binary classification algorithms' performance. The ROC curve is produced by calculating and plotting the true-positive rate against the false-positive rate for a single classifier at various thresholds. The true-positive rate is calculated as the ratio between the number of positive events rightly categorized as positive (true positives) and the total number of actual positive events (regardless of classification, meaning true positives plus false negatives). On the other hand, the false-positive rate is calculated as the ratio between the number of negative events wrongly categorized as positive (false positives) and the total number of actual negative events (regardless of classification, meaning false positives plus true negatives). Overall, the ROC provides a graphical depiction of a classifier's performance rather than a single value like other metrics such as accuracy.

AUC stands for the area under the (ROC) curve. Generally, the higher the AUC score, the better a classifier performs for the given task. The ROC-AUC curve helps us visualize how well our machine learning classifier is performing. In Figures 5-7, the ROC-AUC curve for each method used has been calculated and reported for training, validation, and test groups. The criteria for best model is to have both the prediction strength and generalizability to unseen data, the model should not be overfitted on training data and should have almost equal performance on validation and training and test set. By comparing models' performance for validation and test set, it was concluded that in this study logistic regression has the best total performance and less overfitting to training data. Logistic regression due to its simplicity also has the advantage of powerful explainability compared to other models that are too complex to clearly explain their decision-making process such as multi-layer perceptron and Ensembl random decision tree.

*Second dose of Sputnik V side effect prediction*

The training procedure for the second dose is similar to the first dose except here models have access to the first dose side effect data. As expected, these new features helped the models to have a better prediction for second dose side effects (Figure 7). Except for KNN that showed poor performance on the validation sets, other models showed an average ROC-AUC equal to 0.823.

As some models have been overfitted to training data, the logistic regression has been selected as the best model.

*Extra-validation and generalizability*

To ensure that the models can be used on real world data, 20 percent of initial data has been left unseen from the model in both training and hyperparameter optimization steps so there would not be any information leakage from this test set to the model. By comparing performance of the model on the test set with validation and training sets we can conclude that the model is generalizable enough to use in the real world. In both first and second dose models XGBoost, KNN and RF have been clearly overfitted on training data, while SVM, MLP and LR have comparable performance on training, validation and test set.

*Model-output correlations*

The Logistic regression model coefficient was used to demonstrate each predictor variable's effect on each side effect's outcome. Logistic regression calculates a probability P for in each input data X with following formula:

$$P(X) = \frac{1}{1+e^{-(\beta_0+\beta_1 X_1+\cdots+\beta_i X_i+\cdots+\beta_n X_n)}}$$

where $\beta_i$ is the coefficient for feature i.

The resulting values have been shown in Figure 8 for the first dose and Figure 9 for the second dose of Sputnik V vaccine models, the values for the first dose of AstraZeneca are shown in Figure 10. To compare logistic regression coefficients for different features, all the continuous variables were normalized to avoid undesired upper/under estimation of feature effects on prediction.

## Claims

1. A method for predicting side effects of a coronavirus vaccine in a subject, the method comprising:

(a) providing subject-specific health and life-style data; and
(b) predicting the side effects of a coronavirus vaccine in a subject based on the subject-specific health and

life-style data using a previously trained mathematical model.

2. The method of claim 1, wherein the subject-specific health and life-style data comprise subject physical parameters, course of the past coronavirus disease, data on medication(s) administered to the subject, medical background, and/or life style data, preferably wherein the subject-specific health and life-style data is collected by using a questionnaire.

3. The method of claim 2, wherein the subject physical parameters comprise weight, height, BMI, blood group, age, and/or sex.

4. The method of any one of claims 2 to 3, wherein the course of the past coronavirus disease comprises data on severity, vertigo, sore throat, headache, chest pain, feeling paralyzed, loss of consciousness, breathing difficulties, loss of smell, digestive difficulties, cough, pain, fatigue and/or fever.

5. The method of any one of claims 2 to 4, wherein the medical background comprises the data on pregnancy, allergy, psychological issues, skeletal system, liver diseases, kidney diseases, digestive tract diseases, blood diseases, immune system diseases, lung diseases, neurological diseases, active cancer diseases, past history of cancer disease, hypertension, heart disease, and/or diabetes.

6. The method of any one of claims 2 to 5, wherein the subject-specific health and life-style data further comprise data on side effects of a previous dose of a coronavirus vaccine,
preferably wherein the data on side effects of the previous dose of a coronavirus vaccine comprise data on muscle pain, join paint, chills, nausea, headache, fatigue, fever and/or local side effects.

7. The method of any one of claims 1 to 6, wherein the previously trained mathematical model is a machine learning model,
preferably wherein the machine learning model is selected from Logistic Regression (LR), Random Forest (RF), Multi-Layer Perceptron (MLP), K-Nearest Neighbors (KNN), Support Vector Machine (SVM), and Gradient Boosted Decision Trees (XGBoost).

8. The method of claim 7, wherein the machine learning model is Logistic Regression (LR), preferably wherein the previously trained mathematical model has been trained using hyperparameter tuning.

9. The method of any one of claims 1 to 8, wherein the predicted side effects of administering a vaccine to a subject include fever, fatigue, headache, nausea, chills, joint pain, muscle pain and/or local side effects.

10. The method of any one of claims 1 to 9, wherein the method further comprises the step of (a') training of the previously trained mathematical model.

11. The method of any one of claims 1 to 10, wherein the previously trained mathematical model is trained using the database comprising subject-specific data on side effects of a coronavirus vaccine in a subject and subject-specific health and life-style data, wherein the database has been compiled for subjects that were previously vaccinated with a coronavirus vaccine,

preferably wherein the data on side effects of a coronavirus vaccine in a subject is as described in claim 9, and/or
preferably wherein the subject-specific health and life-style data are as described in any one of claims 2 to 6.

12. The method of any one of claims 1 to 11, wherein the previously trained mathematical model relies on predictors selected from subject-specific health and life-style data, wherein the subject specific health and life-style data are as described in any one of claims 2 to 6.

13. The method of any one of claims 1 to 12, wherein the predicted side effects of a coronavirus vaccine are provided in a form of a subject-specific factsheet.

14. A method for selecting a subject qualifying for vaccination with a coronavirus vaccine, the method comprising

(a) predicting the side effects of a coronavirus vaccine in a subject using the method according to any one of

claims 1 to 13, and
(b) selecting a subject qualifying for vaccination with a coronavirus vaccine based on predicted side effects of a coronavirus vaccine in said subject.

**15.** The method of any one of the preceding claims, wherein the coronavirus is SARS-CoV2.

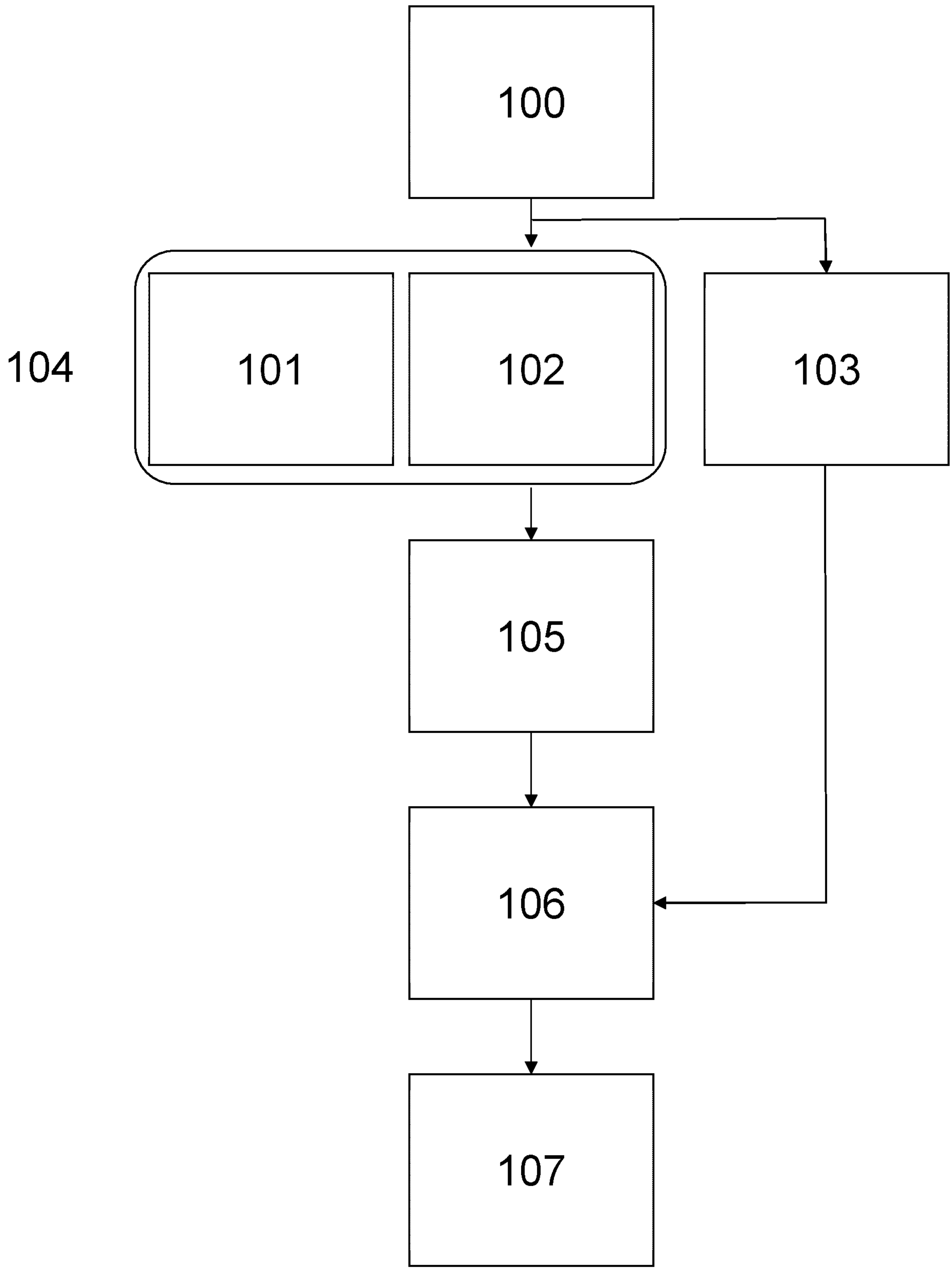


Figure 1.

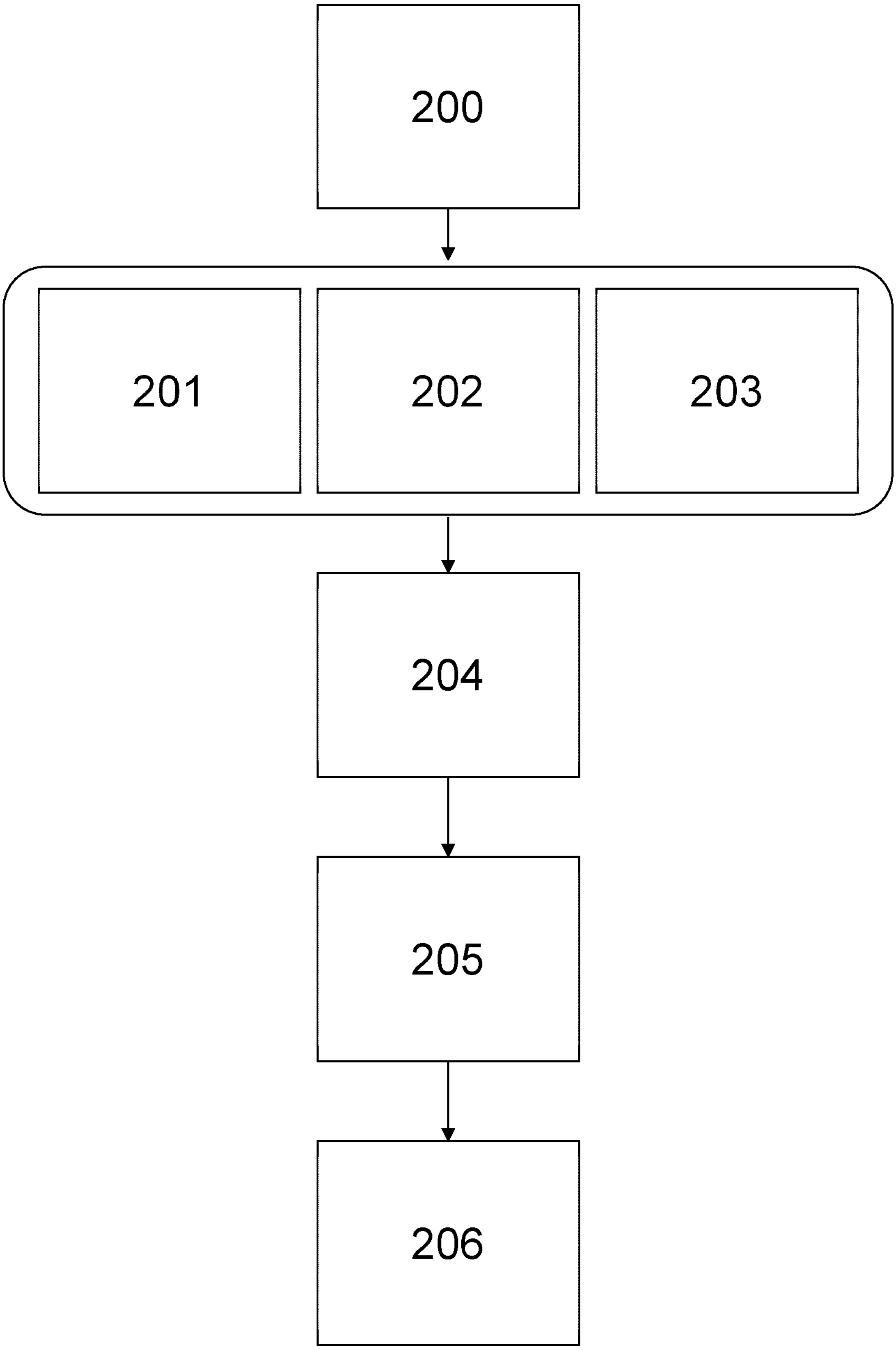


Figure 2.

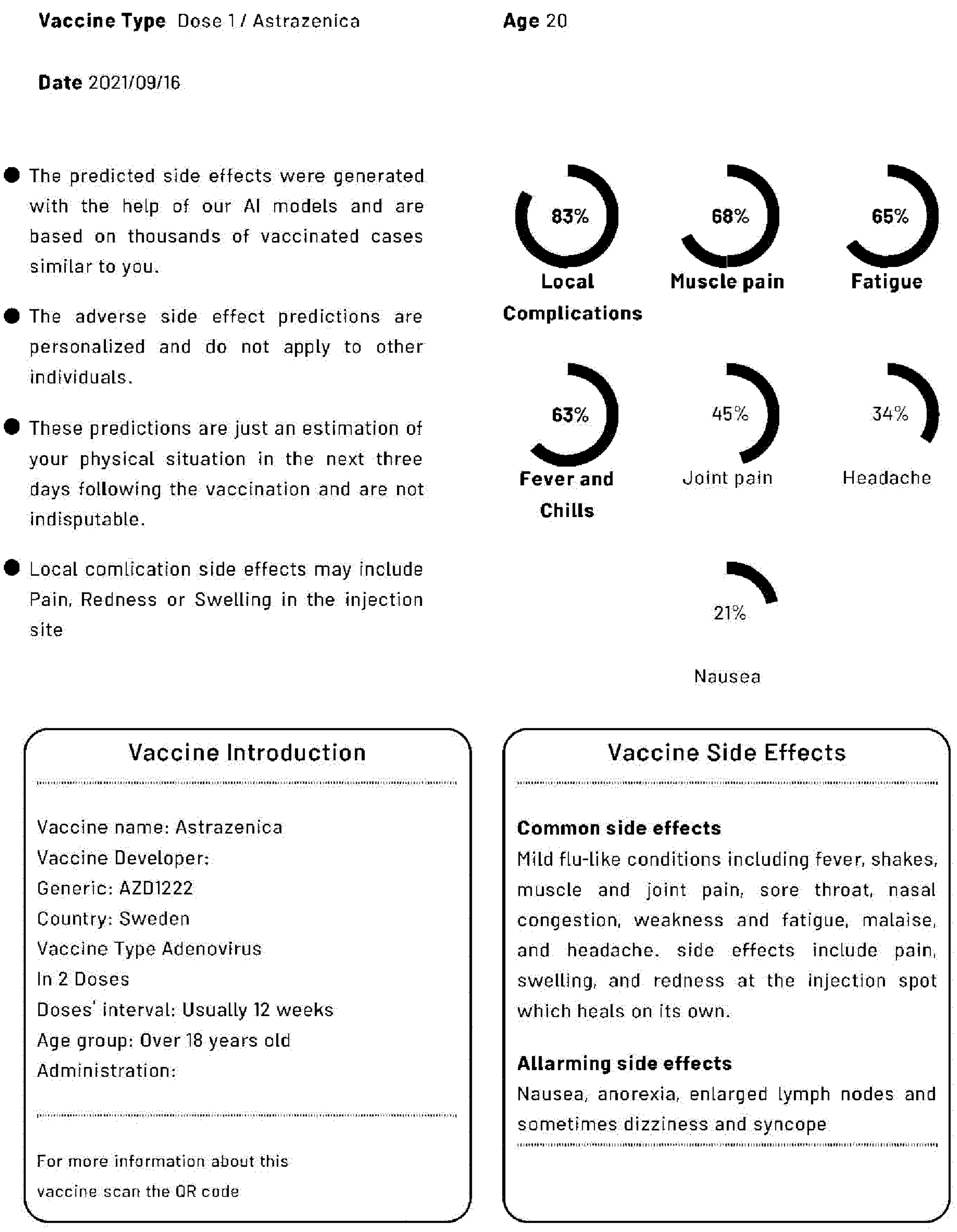
Vaccine Type Dose 1 / Astrazenica
Age 20
Date 2021/09/16
The predicted side effects were generated with the help of our AI models and are based on thousands of vaccinated cases similar to you.
The adverse side effect predictions are personalized and do not apply to other individuals.
These predictions are just an estimation of your physical situation in the next three days following the vaccination and are not indisputable.
Local comlication side effects may include Pain, Redness or Swelling in the injection site
83%
Local Complications
68%
Muscle pain
65%
Fatigue
63%
Fever and Chills
45%
Joint pain
34%
Headache
21%
Nausea
Vaccine Introduction
Vaccine name: Astrazenica
Vaccine Developer:
Generic: AZD1222
Country: Sweden
Vaccine Type Adenovirus
In 2 Doses
Doses' interval: Usually 12 weeks
Age group: Over 18 years old
Administration:
For more information about this vaccine scan the QR code
Vaccine Side Effects
Common side effects
Mild flu-like conditions including fever, shakes, muscle and joint pain, sore throat, nasal congestion, weakness and fatigue, malaise, and headache. side effects include pain, swelling, and redness at the injection spot which heals on its own.
Allarming side effects
Nausea, anorexia, enlarged lymph nodes and sometimes dizziness and syncope

Figure 3.

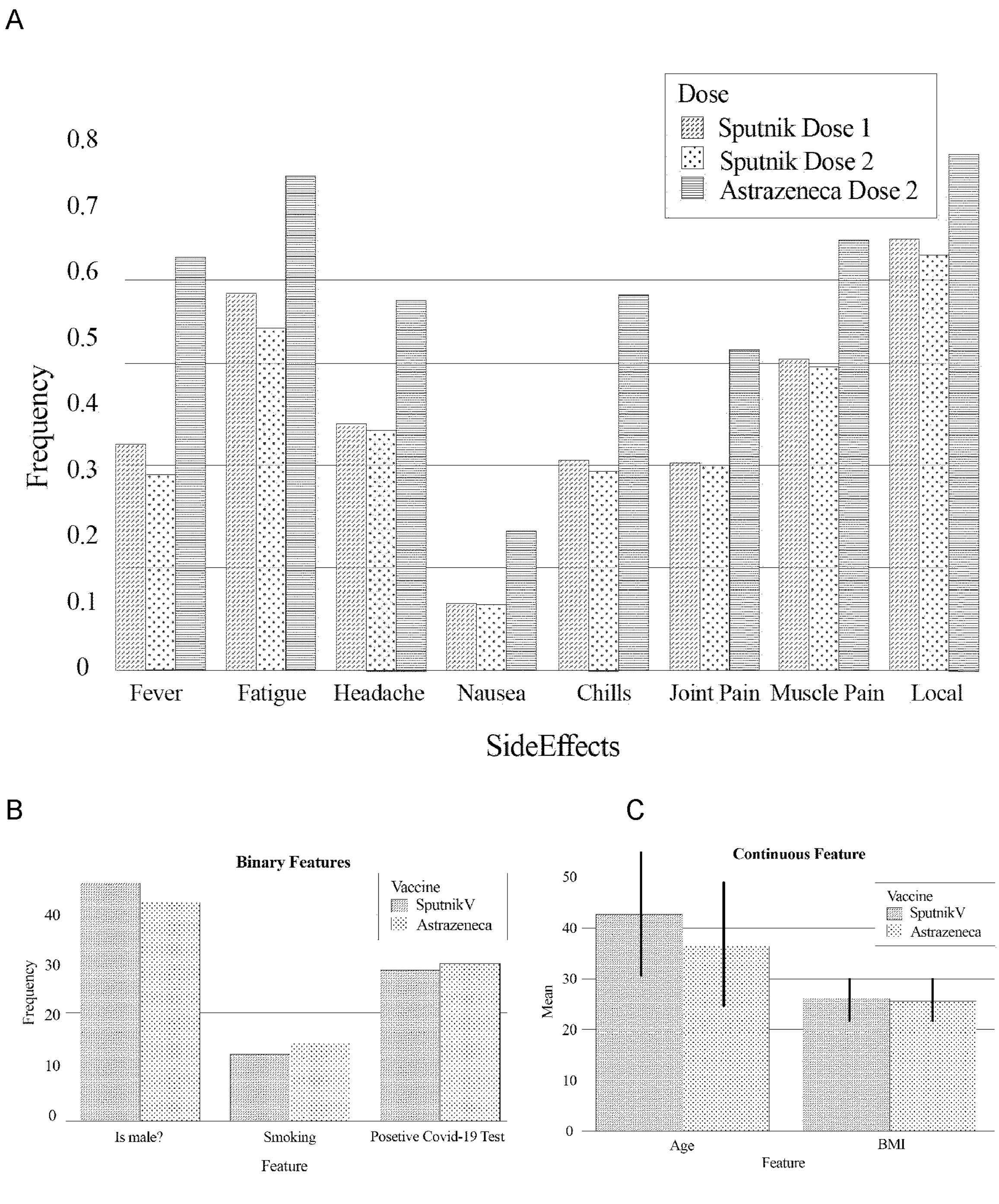
A
Dose
Sputnik Dose 1
Sputnik Dose 2
Astrazeneca Dose 2
Frequency
0
0.1
0.2
0.3
0.4
0.5
0.6
0.7
0.8
Fever
Fatigue
Headache
Nausea
Chills
Joint Pain
Muscle Pain
Local
SideEffects
B
Binary Features
Vaccine
SputnikV
Astrazeneca
Frequency
0
10
20
30
40
Is male?
Smoking
Posetive Covid-19 Test
Feature
C
Continuous Feature
Vaccine
SputnikV
Astrazeneca
Mean
0
10
20
30
40
50
Age
BMI
Feature

Figure 4.

| | | Sputnik V | | | | | | | |
|---|---|---|---|---|---|---|---|---|---|
| | | Fever | Fatigue | Headache | Nausea | Chills | Joint Pain | Muscle Pain | Local |
| Training Set | Logistic Regression | 0.709 | 0.688 | 0.702 | 0.702 | 0.692 | 0.709 | 0.699 | 0.743 |
| | SVM | 0.575 | 0.648 | 0.585 | 0.542 | 0.579 | 0.577 | 0.559 | 0.725 |
| | XGBClassifier | 0.839 | 0.937 | 0.811 | 0.963 | 0.727 | 0.768 | 0.961 | 0.825 |
| | RF | 0.854 | 0.849 | 0.856 | 0.915 | 0.839 | 0.859 | 0.872 | 0.862 |
| | KNN | 1.000 | 1.000 | 1.000 | 1.000 | 1.000 | 1.000 | 1.000 | 1.000 |
| | MLP | 0.675 | 0.681 | 0.688 | 0.630 | 0.646 | 0.680 | 0.682 | 0.738 |
| Validation Set | Logistic Regression | 0.675 | 0.667 | 0.675 | 0.641 | 0.632 | 0.670 | 0.666 | 0.727 |
| | SVM | 0.578 | 0.629 | 0.556 | 0.544 | 0.558 | 0.552 | 0.552 | 0.712 |
| | XGBClassifier | 0.645 | 0.656 | 0.673 | 0.659 | 0.653 | 0.666 | 0.646 | 0.710 |
| | RF | 0.676 | 0.673 | 0.683 | 0.657 | 0.647 | 0.675 | 0.677 | 0.726 |
| | KNN | 0.617 | 0.639 | 0.644 | 0.572 | 0.556 | 0.612 | 0.621 | 0.687 |
| | MLP | 0.650 | 0.663 | 0.667 | 0.616 | 0.621 | 0.655 | 0.656 | 0.723 |
| Test Set | Logistic Regression | 0.630 | 0.666 | 0.675 | 0.710 | 0.655 | 0.675 | 0.666 | 0.737 |
| | SVM | 0.509 | 0.512 | 0.607 | 0.539 | 0.518 | 0.537 | 0.503 | 0.716 |
| | XGBClassifier | 0.609 | 0.654 | 0.665 | 0.691 | 0.614 | 0.632 | 0.645 | 0.735 |
| | RF | 0.638 | 0.677 | 0.677 | 0.692 | 0.663 | 0.671 | 0.676 | 0.738 |
| | KNN | 0.611 | 0.630 | 0.657 | 0.582 | 0.604 | 0.618 | 0.624 | 0.681 |
| | MLP | 0.615 | 0.662 | 0.678 | 0.712 | 0.628 | 0.671 | 0.641 | 0.734 |

Figure 5.

| | | AstraZeneca | | | | | | | |
|---|---|---|---|---|---|---|---|---|---|
| | | Fever | Fatigue | Headache | Nausea | Chills | Joint Pain | Muscle Pain | Local |
| Training Set | Logistic Regression | 0.680 | 0.672 | 0.670 | 0.703 | 0.679 | 0.638 | 0.654 | 0.709 |
| | SVM | 0.679 | 0.629 | 0.668 | 0.580 | 0.679 | 0.626 | 0.650 | 0.651 |
| | XGBClassifier | 0.763 | 0.831 | 0.966 | 0.956 | 0.831 | 0.977 | 0.907 | 0.817 |
| | RF | 0.838 | 0.839 | 0.833 | 0.876 | 0.836 | 0.827 | 0.830 | 0.840 |
| | KNN | 1.000 | 1.000 | 1.000 | 1.000 | 1.000 | 1.000 | 1.000 | 1.000 |
| | MLP | 0.675 | 0.664 | 0.666 | 0.682 | 0.672 | 0.628 | 0.655 | 0.702 |
| Validation Set | Logistic Regression | 0.664 | 0.651 | 0.657 | 0.684 | 0.659 | 0.620 | 0.636 | 0.686 |
| | SVM | 0.664 | 0.608 | 0.651 | 0.573 | 0.658 | 0.610 | 0.632 | 0.650 |
| | XGBClassifier | 0.658 | 0.650 | 0.665 | 0.674 | 0.662 | 0.637 | 0.643 | 0.687 |
| | RF | 0.671 | 0.666 | 0.668 | 0.686 | 0.671 | 0.634 | 0.656 | 0.695 |
| | KNN | 0.638 | 0.648 | 0.638 | 0.651 | 0.638 | 0.616 | 0.649 | 0.656 |
| | MLP | 0.658 | 0.647 | 0.655 | 0.679 | 0.654 | 0.616 | 0.635 | 0.681 |
| Test Set | Logistic Regression | 0.666 | 0.654 | 0.680 | 0.709 | 0.656 | 0.620 | 0.657 | 0.667 |
| | SVM | 0.667 | 0.642 | 0.675 | 0.499 | 0.655 | 0.592 | 0.649 | 0.607 |
| | XGBClassifier | 0.683 | 0.653 | 0.668 | 0.706 | 0.648 | 0.636 | 0.632 | 0.698 |
| | RF | 0.681 | 0.662 | 0.696 | 0.727 | 0.662 | 0.640 | 0.660 | 0.694 |
| | KNN | 0.667 | 0.661 | 0.688 | 0.701 | 0.654 | 0.622 | 0.636 | 0.667 |
| | MLP | 0.667 | 0.648 | 0.675 | 0.704 | 0.655 | 0.613 | 0.658 | 0.668 |

Figure 6.

| | | Sputnik V | | | | | | | |
|---|---|---|---|---|---|---|---|---|---|
| | | Fever | Fatigue | Headache | Nausea | Chills | Joint Pain | Muscle Pain | Local |
| Training Set | Logistic Regression | 0.923 | 0.907 | 0.905 | 0.888 | 0.889 | 0.908 | 0.901 | 0.899 |
| | SVM | 0.885 | 0.883 | 0.874 | 0.860 | 0.838 | 0.856 | 0.873 | 0.874 |
| | XGBClassifier | 0.975 | 0.959 | 0.975 | 0.901 | 0.994 | 0.916 | 0.951 | 0.960 |
| | RF | 0.977 | 0.966 | 0.960 | 0.985 | 0.990 | 0.967 | 0.936 | 0.955 |
| | KNN | 1.000 | 1.000 | 1.000 | 1.000 | 1.000 | 1.000 | 1.000 | 1.000 |
| | MLP | 0.923 | 0.912 | 0.910 | 0.869 | 0.882 | 0.901 | 0.905 | 0.910 |
| Validation Set | Logistic Regression | 0.906 | 0.903 | 0.901 | 0.857 | 0.861 | 0.887 | 0.895 | 0.894 |
| | SVM | 0.870 | 0.874 | 0.861 | 0.816 | 0.808 | 0.832 | 0.865 | 0.874 |
| | XGBClassifier | 0.902 | 0.890 | 0.902 | 0.844 | 0.841 | 0.879 | 0.882 | 0.893 |
| | RF | 0.912 | 0.903 | 0.907 | 0.868 | 0.859 | 0.892 | 0.897 | 0.901 |
| | KNN | 0.833 | 0.858 | 0.865 | 0.659 | 0.728 | 0.825 | 0.841 | 0.869 |
| | MLP | 0.906 | 0.902 | 0.901 | 0.857 | 0.860 | 0.889 | 0.895 | 0.896 |
| Test Set | Logistic Regression | 0.860 | 0.890 | 0.902 | 0.842 | 0.794 | 0.895 | 0.910 | 0.893 |
| | SVM | 0.867 | 0.873 | 0.859 | 0.806 | 0.769 | 0.832 | 0.881 | 0.841 |
| | XGBClassifier | 0.867 | 0.881 | 0.889 | 0.845 | 0.774 | 0.874 | 0.904 | 0.881 |
| | RF | 0.873 | 0.895 | 0.904 | 0.813 | 0.793 | 0.892 | 0.909 | 0.892 |
| | KNN | 0.813 | 0.872 | 0.850 | 0.688 | 0.682 | 0.826 | 0.852 | 0.875 |
| | MLP | 0.857 | 0.887 | 0.892 | 0.843 | 0.759 | 0.909 | 0.907 | 0.888 |

Figure 7.

| | Sputnik V - Dose 1 | | | | | | | |
|---|---|---|---|---|---|---|---|---|
| | Fever | Fatigue | Headache | Nausea | Chills | Joint Pain | Muscle Pain | Local |
| Sex | -0.251 | -0.411 | -0.363 | -0.964 | -0.207 | -0.211 | -0.203 | -0.890 |
| Age | -4.277 | -2.660 | -2.614 | -2.026 | -4.895 | -4.156 | -3.651 | -2.087 |
| A | 0.126 | 0.262 | 0.014 | -0.033 | 0.126 | -0.019 | 0.019 | 0.068 |
| AB | -0.147 | 0.053 | 0.079 | 0.082 | 0.018 | 0.073 | -0.047 | -0.111 |
| B | -0.018 | 0.132 | 0.018 | -0.134 | -0.082 | -0.058 | -0.017 | 0.007 |
| O | 0.044 | 0.234 | 0.106 | 0.085 | 0.131 | 0.007 | 0.040 | 0.037 |
| Smoking | 0.014 | 0.072 | -0.199 | 0.158 | 0.148 | 0.123 | -0.019 | -0.019 |
| Substance use | 0.248 | 0.461 | -0.239 | -0.356 | -0.822 | 0.228 | 0.719 | -0.084 |
| Alcohol dependancy | -0.085 | -0.118 | 0.079 | 0.291 | 0.441 | 0.034 | 0.046 | 0.353 |
| Background Diabetes | -0.074 | -0.269 | -0.057 | -0.061 | -0.145 | 0.011 | -0.248 | -0.041 |
| Background Cardiovascular disease | 0.098 | -0.092 | -0.023 | -0.244 | 0.009 | 0.005 | 0.054 | -0.042 |
| Background Hypertension | 0.147 | 0.223 | -0.096 | 0.087 | -0.104 | 0.197 | 0.164 | 0.204 |
| Background CancerPassive | 0.263 | -0.166 | -0.198 | 0.306 | 0.424 | 0.512 | -0.207 | -0.147 |
| Background CancerActive | -0.132 | 0.057 | 0.256 | 0.216 | 0.947 | 0.602 | 0.400 | -0.111 |
| Background Neurological | 0.063 | 0.579 | -0.097 | 0.490 | 0.309 | 0.247 | 0.270 | -0.076 |
| Background pulmonary | 0.372 | -0.096 | 0.058 | 0.374 | -0.034 | 0.023 | 0.075 | -0.145 |
| Background ImmuneSystem | 0.066 | 0.173 | -0.221 | -0.040 | 0.323 | 0.173 | 0.178 | 0.117 |
| Background hematologic | 0.384 | -0.116 | -0.033 | -0.004 | 0.048 | 0.304 | -0.183 | -0.109 |
| Background Gastrointestinal | 0.339 | 0.407 | 0.395 | 0.461 | 0.463 | 0.346 | 0.411 | 0.183 |
| Background Renal | 0.596 | 0.024 | 0.091 | 0.382 | 0.386 | 0.591 | 0.289 | 0.158 |
| Background Hepatic | 0.209 | -0.045 | -0.139 | 0.174 | -0.278 | -0.149 | -0.227 | -0.313 |
| Background Skeletal | 0.249 | 0.200 | 0.114 | 0.161 | 0.241 | 0.516 | 0.283 | 0.167 |
| Background Mental | -0.113 | -0.038 | -0.215 | -0.541 | -0.240 | 0.134 | -0.046 | -0.220 |
| Background Allergy | 0.266 | 0.263 | 0.056 | 0.146 | 0.292 | 0.149 | 0.206 | 0.244 |
| Background None | 0.145 | 0.065 | -0.101 | 0.021 | 0.096 | 0.069 | 0.027 | 0.056 |
| Hormonal med | 0.049 | 0.396 | 0.568 | 0.446 | 0.192 | 0.052 | 0.371 | 0.218 |
| Pregnancy | 0.028 | -0.023 | -0.043 | -0.248 | 0.175 | 0.069 | 0.238 | -0.122 |
| Respiratory Inhaler | -0.088 | 0.119 | 0.231 | 0.403 | -0.036 | 0.169 | -0.094 | 0.052 |
| Corticosteroid Med | 0.025 | 0.053 | -0.095 | 0.119 | 0.020 | -0.086 | 0.002 | 0.023 |
| Chemotherapy Med | -0.223 | -0.259 | -0.129 | -1.309 | -0.201 | -0.100 | -0.060 | -0.274 |
| Immunosuppressive Med | -0.082 | -0.079 | -0.272 | 0.316 | 0.031 | 0.007 | -0.079 | -0.305 |
| Covid Infection | -0.591 | -0.839 | -0.875 | -0.812 | -0.683 | -0.954 | -0.884 | -0.263 |
| Covid Fever | 0.985 | 0.197 | 0.167 | 0.033 | 0.907 | 0.349 | 0.219 | 0.189 |
| Covid Fatigue | 0.198 | 0.950 | 0.240 | 0.099 | 0.295 | 0.755 | 1.008 | 0.299 |
| Covid Cough | -0.039 | -0.109 | 0.175 | -0.041 | 0.157 | -0.142 | 0.004 | -0.133 |
| Covid Gastrointestinal | 0.048 | 0.314 | 0.080 | 0.474 | -0.028 | 0.171 | 0.333 | 0.105 |
| Covid Anosmia | -0.036 | 0.088 | -0.102 | 0.081 | -0.008 | 0.107 | 0.126 | 0.113 |
| Covid RespiratoryProblems | 0.096 | 0.064 | -0.050 | 0.003 | -0.092 | 0.091 | -0.023 | 0.344 |
| Covid ConsciousnessDisorder | 0.510 | 0.216 | 0.604 | 0.355 | 0.147 | 0.760 | 0.075 | -0.132 |
| Covid Paresis | -0.058 | 0.294 | 0.524 | 0.286 | 0.168 | 0.811 | 0.412 | -0.150 |
| Covid ChestPain | -0.053 | 0.312 | 0.092 | 0.121 | -0.025 | 0.280 | 0.250 | 0.218 |
| Covid Headache | 0.172 | 0.328 | 1.193 | 0.593 | 0.184 | 0.317 | 0.263 | 0.368 |
| Covid SoreThroat | -0.147 | -0.317 | -0.163 | -0.212 | -0.335 | -0.196 | -0.221 | -0.148 |
| Covid Vertigo | 0.307 | 0.173 | 0.402 | 0.578 | 0.309 | 0.350 | 0.294 | -0.003 |
| Covid hospitalization | 0.207 | 0.004 | 0.003 | 0.368 | 0.000 | -0.147 | -0.273 | 0.119 |
| BMI | -0.542 | 0.127 | -0.080 | -0.135 | -2.124 | -0.298 | -0.111 | 0.007 |

Figure 8.

| | Sputnik V - Dose 2 | | | | | | | |
|---|---|---|---|---|---|---|---|---|
| | Fever | Fatigue | Headache | Nausea | Chills | Joint Pain | Muscle Pain | Local |
| Sex | 0.071 | 0.053 | -0.180 | -0.541 | -0.150 | -0.151 | -0.043 | -0.412 |
| Age | -0.492 | -0.205 | -0.280 | -0.565 | -0.549 | -0.652 | -0.072 | -0.169 |
| A | -0.384 | -0.123 | -0.403 | -0.587 | -0.421 | -0.340 | -0.048 | -0.054 |
| AB | -0.328 | -0.063 | -0.260 | -0.243 | -0.349 | -0.289 | -0.001 | -0.032 |
| B | -0.477 | 0.045 | -0.382 | -0.497 | -0.307 | -0.365 | -0.039 | 0.043 |
| O | -0.374 | 0.142 | -0.324 | -0.399 | -0.282 | -0.350 | 0.088 | 0.044 |
| Smoking | 0.036 | 0.037 | 0.033 | -0.221 | 0.084 | -0.065 | -0.031 | 0.100 |
| Substance use | 0.314 | 0.072 | 0.066 | 0.023 | -0.092 | 0.055 | 0.011 | -0.077 |
| Alcohol dependancy | 0.746 | 0.069 | 0.028 | 0.114 | 0.243 | 0.116 | 0.031 | 0.021 |
| Background Diabetes | -0.041 | -0.115 | 0.032 | -0.366 | -0.111 | -0.208 | -0.064 | 0.135 |
| Background Cardiovascular disease | -0.298 | 0.045 | -0.121 | -0.047 | -0.143 | -0.276 | -0.004 | -0.331 |
| Background Hypertension | 0.181 | -0.058 | -0.078 | -0.273 | 0.059 | -0.083 | -0.048 | 0.101 |
| Background CancerPassive | -0.241 | 0.193 | 0.134 | -0.287 | -0.205 | -0.095 | -0.017 | 0.132 |
| Background CancerActive | -0.279 | -0.025 | 0.033 | -0.050 | 0.095 | -0.038 | 0.003 | -0.039 |
| Background Neurological | 0.102 | -0.072 | 0.017 | -0.282 | 0.068 | -0.065 | 0.045 | -0.087 |
| Background pulmonary | 0.249 | 0.172 | 0.242 | -0.003 | 0.090 | 0.227 | 0.056 | -0.273 |
| Background ImmuneSystem | -0.028 | 0.173 | 0.021 | 0.116 | 0.088 | -0.115 | 0.035 | 0.208 |
| Background hematologic | 0.747 | -0.077 | 0.105 | 0.077 | 0.268 | 0.035 | 0.025 | 0.224 |
| Background Gastrointestinal | -0.053 | 0.105 | -0.103 | -0.104 | 0.012 | 0.035 | 0.056 | 0.295 |
| Background Renal | 0.301 | 0.257 | 0.132 | 0.045 | 0.093 | 0.240 | 0.007 | 0.088 |
| Background Hepatic | 0.065 | -0.004 | -0.077 | -0.024 | 0.080 | -0.105 | 0.004 | -0.077 |
| Background Skeletal | -0.017 | -0.012 | -0.139 | 0.079 | -0.143 | 0.238 | 0.031 | -0.190 |
| Background Mental | 0.792 | 0.318 | 0.083 | 0.007 | 0.235 | 0.183 | 0.056 | 0.113 |
| Background Allergy | 0.226 | 0.010 | 0.043 | -0.092 | -0.178 | -0.042 | 0.061 | 0.133 |
| Background None | -0.006 | -0.120 | -0.194 | -0.352 | -0.079 | -0.251 | -0.052 | -0.111 |
| Hormonal med | 0.460 | 0.365 | -0.099 | -0.053 | 0.363 | 0.313 | 0.037 | 0.288 |
| Pregnancy | 0.431 | 0.379 | 0.115 | 0.220 | 0.244 | 0.326 | 0.113 | 0.118 |
| Respiratory Inhaler | -0.247 | -0.152 | -0.151 | -0.053 | -0.157 | -0.166 | -0.032 | 0.021 |
| Corticosteroid Med | 0.103 | 0.107 | -0.204 | -0.305 | -0.092 | -0.092 | -0.007 | -0.050 |
| Chemotherapy Med | 0.201 | -0.143 | 0.129 | 0.115 | 0.165 | 0.128 | -0.021 | -0.189 |
| Immunosuppressive Med | -0.286 | -0.042 | 0.111 | -0.047 | -0.042 | 0.020 | -0.029 | 0.027 |
| Covid Infection | -0.232 | -0.192 | -0.221 | -0.421 | -0.065 | -0.310 | -0.069 | -0.302 |
| Covid Fever | 0.237 | -0.131 | -0.071 | 0.130 | 0.180 | -0.074 | 0.019 | -0.111 |
| Covid Fatigue | -0.060 | 0.434 | 0.054 | -0.243 | 0.071 | 0.335 | 0.158 | 0.275 |
| Covid Cough | 0.012 | 0.003 | -0.127 | -0.101 | -0.014 | -0.077 | 0.024 | 0.042 |
| Covid Gastrointestinal | 0.281 | 0.063 | -0.190 | 0.341 | 0.076 | 0.076 | 0.026 | -0.036 |
| Covid Anosmia | 0.026 | 0.079 | -0.072 | 0.271 | -0.048 | -0.061 | -0.024 | 0.117 |
| Covid RespiratoryProblems | 0.090 | 0.136 | 0.092 | 0.141 | 0.210 | 0.139 | 0.023 | 0.112 |
| Covid ConsciousnessDisorder | -0.024 | 0.105 | -0.052 | -0.129 | 0.099 | 0.144 | -0.023 | -0.083 |
| Covid Paresis | -0.133 | -0.087 | -0.119 | 0.074 | 0.027 | 0.131 | 0.015 | -0.006 |
| Covid ChestPain | 0.109 | -0.112 | 0.125 | 0.292 | -0.123 | 0.366 | 0.047 | 0.140 |
| Covid Headache | 0.056 | 0.093 | 0.814 | 0.037 | 0.002 | 0.024 | -0.010 | 0.094 |
| Covid SoreThroat | -0.091 | 0.013 | -0.090 | -0.067 | -0.017 | -0.059 | 0.013 | 0.162 |
| Covid Vertigo | -0.087 | 0.240 | 0.305 | 0.286 | -0.077 | 0.118 | 0.062 | -0.123 |
| Covid hospitalization | 0.102 | -0.126 | -0.353 | 0.061 | 0.111 | -0.148 | -0.028 | -0.031 |
| Dose1_Fever | 2.776 | -0.044 | 0.191 | 0.121 | 0.105 | -0.281 | 0.145 | -0.004 |
| Dose1_Fatigue | 0.051 | 2.269 | 0.326 | -0.168 | 0.070 | 0.230 | 0.449 | 0.266 |
| Dose1_Headache | 0.122 | 0.435 | 2.587 | 0.368 | 0.098 | 0.221 | 0.298 | 0.153 |
| Dose1_Nausea | 0.272 | 0.237 | 0.247 | 2.928 | 0.011 | 0.217 | 0.079 | -0.211 |
| Dose1_Chills | -0.060 | -0.125 | -0.097 | 0.079 | 2.217 | -0.062 | 0.089 | -0.144 |
| Dose1_Joint_Pain | 0.054 | 0.122 | -0.013 | 0.181 | 0.181 | 2.582 | 0.489 | 0.023 |
| Dose1_Muscle_Pain | -0.159 | 0.439 | 0.126 | 0.064 | 0.005 | 0.483 | 1.445 | 0.069 |
| Dose1_Local | 0.105 | 0.032 | 0.012 | -0.158 | 0.074 | -0.150 | 0.099 | 2.750 |
| BMI | -0.925 | -0.364 | -0.259 | -0.345 | -0.449 | -0.150 | -0.008 | -0.143 |

Figure 9.

| | Astrazeneca - Dose 1 | | | | | | | |
|---|---|---|---|---|---|---|---|---|
| | Fever | Fatigue | Headache | Nausea | Chills | Joint Pain | Muscle Pain | Local |
| Sex | -0.242 | -0.310 | -0.527 | -1.056 | -0.275 | -0.250 | -0.317 | -0.796 |
| Age | -3.380 | -2.618 | -1.503 | -1.187 | -2.869 | -2.162 | -1.935 | -2.582 |
| A | 0.026 | -0.059 | 0.143 | -0.192 | -0.030 | -0.002 | -0.087 | 0.014 |
| AB | 0.051 | 0.023 | 0.206 | -0.013 | 0.039 | 0.183 | 0.165 | 0.039 |
| B | -0.029 | 0.082 | 0.018 | -0.240 | 0.000 | -0.064 | 0.002 | -0.031 |
| O | -0.047 | -0.044 | 0.027 | -0.261 | -0.007 | -0.020 | -0.080 | -0.022 |
| Smoking | 0.201 | 0.304 | -0.058 | 0.202 | 0.134 | 0.155 | 0.281 | -0.029 |
| Substance use | -0.320 | -0.001 | -0.073 | -0.115 | -0.293 | 0.048 | 0.040 | -0.239 |
| Alcohol dependancy | -0.134 | 0.015 | -0.056 | 0.146 | 0.141 | 0.420 | 0.200 | 0.424 |
| Background Diabetes | 0.300 | 0.097 | 0.073 | 0.221 | -0.162 | -0.018 | 0.046 | 0.060 |
| Background Cardiovascular disease | -0.131 | -0.062 | 0.008 | 0.069 | -0.065 | 0.052 | -0.054 | -0.214 |
| Background Hypertension | 0.304 | 0.239 | -0.011 | -0.208 | 0.184 | 0.146 | 0.054 | 0.264 |
| Background CancerPassive | 0.427 | 0.457 | 0.185 | 0.163 | 0.024 | 0.214 | 0.324 | 0.302 |
| Background CancerActive | -0.393 | 0.225 | -0.019 | 0.081 | 0.037 | -0.369 | -0.032 | 0.533 |
| Background Neurological | 0.218 | 0.194 | 0.052 | 0.262 | 0.113 | 0.226 | 0.195 | -0.116 |
| Background pulmonary | 0.144 | 0.275 | 0.257 | -0.121 | 0.245 | 0.204 | 0.212 | 0.286 |
| Background ImmuneSystem | 0.063 | 0.277 | -0.020 | -0.150 | -0.143 | 0.411 | 0.266 | 0.140 |
| Background hematologic | -0.141 | 0.054 | 0.387 | 0.047 | 0.623 | 0.162 | -0.068 | 0.237 |
| Background Gastrointestinal | 0.140 | 0.435 | 0.301 | 0.276 | 0.410 | 0.321 | 0.144 | -0.164 |
| Background Renal | 0.246 | -0.038 | -0.043 | -0.162 | 0.493 | 0.215 | -0.002 | 0.285 |
| Background Hepatic | -0.194 | -0.217 | -0.042 | -0.250 | 0.233 | 0.269 | 0.039 | 0.338 |
| Background Skeletal | 0.113 | 0.057 | 0.155 | -0.149 | 0.120 | 0.408 | 0.173 | 0.085 |
| Background Mental | -0.073 | -0.460 | 0.157 | -0.043 | 0.161 | -0.195 | -0.073 | -0.221 |
| Background Allergy | 0.196 | 0.375 | 0.236 | 0.152 | 0.438 | 0.435 | 0.291 | 0.277 |
| Background None | 0.016 | 0.066 | 0.089 | 0.012 | 0.300 | 0.242 | 0.124 | 0.148 |
| Hormonal med | 0.077 | 0.193 | -0.021 | -0.121 | 0.043 | 0.053 | 0.157 | 0.474 |
| Pregnancy | 0.053 | 0.106 | 0.073 | -0.070 | 0.052 | -0.041 | -0.029 | 0.024 |
| Respiratory Inhaler | 0.009 | -0.076 | -0.025 | -0.085 | -0.199 | -0.151 | -0.005 | -0.138 |
| Corticosteroid Med | -0.076 | 0.178 | -0.153 | 0.262 | 0.369 | 0.072 | 0.030 | 0.341 |
| Chemotherapy Med | -0.018 | -0.237 | 0.063 | -0.003 | -0.201 | -0.177 | -0.243 | -0.162 |
| Immunosuppressive Med | -0.307 | -0.189 | -0.133 | -0.366 | -0.268 | -0.018 | -0.235 | -0.216 |
| Covid Infection | -0.588 | -1.017 | -0.704 | -0.461 | -0.518 | -0.572 | -0.567 | 0.016 |
| Covid Fever | 0.965 | 0.102 | -0.002 | 0.141 | 0.828 | -0.041 | 0.049 | -0.058 |
| Covid Fatigue | 0.017 | 0.836 | 0.001 | -0.084 | -0.067 | 0.446 | 0.527 | 0.081 |
| Covid Cough | 0.209 | -0.076 | 0.089 | 0.053 | -0.036 | 0.150 | 0.031 | 0.054 |
| Covid Gastrointestinal | 0.226 | 0.332 | 0.099 | 0.417 | 0.113 | 0.247 | 0.268 | 0.418 |
| Covid Anosmia | 0.040 | -0.051 | -0.096 | -0.010 | 0.125 | 0.089 | -0.021 | -0.001 |
| Covid RespiratoryProblems | -0.012 | 0.362 | 0.095 | -0.029 | -0.023 | 0.291 | 0.241 | 0.219 |
| Covid ConsciousnessDisorder | 0.372 | -0.071 | 0.190 | 0.488 | 0.258 | -0.039 | -0.174 | -0.056 |
| Covid Paresis | -0.349 | 0.145 | -0.064 | 0.050 | -0.228 | 0.384 | 0.092 | -0.229 |
| Covid ChestPain | 0.104 | -0.145 | 0.135 | 0.172 | 0.005 | 0.174 | 0.080 | 0.074 |
| Covid Headache | -0.043 | 0.186 | 0.933 | 0.181 | -0.085 | -0.019 | 0.138 | 0.060 |
| Covid SoreThroat | -0.279 | 0.160 | 0.133 | 0.009 | 0.088 | 0.068 | -0.058 | -0.026 |
| Covid Vertigo | 0.278 | 0.117 | 0.439 | 0.502 | 0.379 | 0.337 | 0.307 | -0.145 |
| Covid hospitalization | 0.014 | -0.059 | -0.097 | 0.060 | -0.177 | -0.169 | -0.235 | 0.021 |
| BMI | -1.717 | -0.767 | -0.141 | -0.304 | -0.961 | -0.139 | -0.151 | -0.871 |

Figure 10.

Europäisches Patentamt
European Patent Office
Office européen des brevets

# EUROPEAN SEARCH REPORT

Application Number
EP 21 20 1799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 127 506 B1 (JAIN PRADUMAN [US] ET AL) 21 September 2021 (2021-09-21)<br>* column 46, lines 20-27 *<br>* column 46, lines 5-11 *<br>----- | 1-15 | INV.<br>G16H10/20<br>G16H50/20<br>G16H50/70 |
| A | BADILLO SOLVEIG ET AL: "An Introduction to Machine Learning",<br>CLINICAL PHARMACOLOGY AND THERAPEUTICS,<br>vol. 107, no. 4, 3 March 2020 (2020-03-03)<br>, pages 871-885, XP055791626,<br>US<br>ISSN: 0009-9236, DOI: 10.1002/cpt.1796<br>* Section: Supervised Learning *<br>----- | 1-15 | |

TECHNICAL FIELDS SEARCHED (IPC)

G16H

2

The present search report has been drawn up for all claims

| Place of search | Date of completion of the search | Examiner |
|---|---|---|
| Munich | 4 March 2022 | Samulowitz, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 20 1799

This annex lists the patent family members relating to the patent documents cited in the above-mentioned European search report. The members are as contained in the European Patent Office EDP file on
The European Patent Office is in no way liable for these particulars which are merely given for the purpose of information.

04-03-2022

| Patent document cited in search report | Publication date | Patent family member(s) | Publication date |
| --- | --- | --- | --- |
| US 11127506 B1 | 21-09-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- AU 2020102250
- AU 2020101336
- EP 2601197 C2
- US 9235686 B
- CN 111768873 A


### Non-patent literature cited in the description


- *WHO Director-General's opening remarks at the media briefing on COVID-19,* 11 March 2020, https://www.who.int/director-general/speeches/detail/who-director-general-s-opening-remarks-at-the-media-briefing-on-covid-19---11-march-2020
- *No winners but fewer losers in global economy from COVID than expected,* 17 May 2021, https://news.un.org/en/story/2021/03/1087712
- **NEJADGHADERI SA ; SAGHAZADEH A ; REZAEI N.** Health Care Policies and COVID-19 Prevalence: Is There Any Association?. *Int J Health Serv,* 2021
- *Diagnostics laboratory emergency use listing,* 17 May 2021, https://www.who.int/teams/regulation-prequalification/eul
- *Allergic Reactions Including Anaphylaxis After Receipt of the First Dose of Moderna COVID-19 Vaccine - United States,* 21 December 2020, vol. 70, 125-129
- **RIAD A ; POKORNÁ A ; ATTIA S ; KLUGAROVÁ J ; KOŠČÍK M ; KLUGAR M.** Prevalence of COVID-19 Vaccine Side Effects among Healthcare Workers in the Czech Republic. *J Clin Med Res,* 2021, https://doi.org/10.3390/jcm10071428
- **KLEIN SL ; MARRIOTT I ; FISH EN.** Sex-based differences in immune function and responses to vaccination. *Trans R Soc Trop Med Hyg,* 2015, vol. 109, 9-15
- **KOPSAFTIS Z ; WOOD-BAKER R ; POOLE P.** Influenza vaccine for chronic obstructive pulmonary disease (COPD). *Cochrane Database Syst Rev,* 2018, vol. 6, CD002733
- **FEKRVAND S ; YAZDANI R ; OLBRICH P et al.** Primary Immunodeficiency Diseases and Bacillus Calmette-Guérin (BCG)-Vaccine-Derived Complications: A Systematic Review. *J Allergy Clin Immunol Pract,* 2020, vol. 8, 1371-1386
- **GARRETA, R. ; MONCECCHI, G.** Learning scikit-learn: Machine Learning in Python. Packt Publishing, 2013